(19) Europäisches Patentamt
European Patent Office
Office européen des brevets

(11) EP 1 485 064 B1

(12) **FASCICULE DE BREVET EUROPEEN**

(45) Date de publication et mention de la délivrance du brevet:
**07.05.2014 Bulletin 2014/19**

(21) Numéro de dépôt: **03744477.5**

(22) Date de dépôt: **20.03.2003**

(51) Int Cl.:
***A61K 8/97*** *(2006.01)* ***A61Q 5/00*** *(2006.01)*
***A61Q 19/00*** *(2006.01)* ***A61K 36/05*** *(2006.01)*
***A61K 36/13*** *(2006.01)* ***A61K 36/16*** *(2006.01)*
***A61K 36/185*** *(2006.01)* ***A61K 36/24*** *(2006.01)*
***A61K 36/30*** *(2006.01)* ***A61K 36/328*** *(2006.01)*
***A61K 36/48*** *(2006.01)* ***A61K 36/487*** *(2006.01)*
***A61K 36/53*** *(2006.01)* ***A61K 36/537*** *(2006.01)*
***A61K 36/61*** *(2006.01)* ***A61K 36/66*** *(2006.01)*
***A61K 36/87*** *(2006.01)* ***A61K 36/88*** *(2006.01)*
***A61K 36/896*** *(2006.01)* ***A61K 36/8965*** *(2006.01)*

(86) Numéro de dépôt international:
**PCT/IB2003/001020**

(87) Numéro de publication internationale:
**WO 2003/077881 (25.09.2003 Gazette 2003/39)**

(54) **Composition cosmétique contenant de phytoalexines et procede d'obtention de phytoalexines**

Phytoalexinen enthaltende kosmetische Zusammensetzung und Phytoalexinen Herstellungsverfahren

Cosmetic composition comprising phytoalexines and method for production of phytoalexins

(84) Etats contractants désignés:
**AT BE BG CH CY CZ DE DK EE ES FI FR GB GR HU IE IT LI LU MC NL PT RO SE SI SK TR**

(30) Priorité: **20.03.2002 FR 0203423**
**26.09.2002 PCT/IB02/03971**

(43) Date de publication de la demande:
**15.12.2004 Bulletin 2004/51**

(73) Titulaire: **Ennamany, Rachid**
**33140 Villenave d'Ornon (FR)**

(72) Inventeur: **Ennamany, Rachid**
**33140 Villenave d'Ornon (FR)**

(74) Mandataire: **Powis de Tenbossche, Roland et al**
**Cabinet Bede S.A.**
**Boulevard Général Wahis 15**
**1030 Bruxelles (BE)**

(56) Documents cités:
**EP-A- 0 378 921** **EP-A- 1 203 811**
**FR-A- 2 795 637** **FR-A- 2 795 965**
**FR-A- 2 808 190**

- **PATENT ABSTRACTS OF JAPAN vol. 1999, no. 05 (C, & JP 11 046782 A (MITSUI CHEM INC), 23 février 1999 (1999-02-23)**
- **PATENT ABSTRACTS OF JAPAN vol. 2000, no. 21 (C, & JP 2001 114619 A (ST LOUIS INTERNATIONAL INC), 24 avril 2001 (2001-04-24)**
- **DATABASE MEDLINE [Online] US NATIONAL LIBRARY OF MEDICINE (NLM), BETHESDA, MD, US; octobre 1999 (1999-10), DOUILLET-BREUIL ET AL.: "Changes in the phytoalexin content of various Vitis spp. in response to ultraviolet C elicitation" XP002231068 Database accession no. NLM10552833**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet une composition à usage topique, en particulier cosmétique, riche en métabolites produits par des cellules végétales dédifférentiées. En particulier, l'invention a pour objet une composition contenant un broyat de cellules végétales dédifférenciées et élicitées en culture in vitro.

Par cellules végétales dédifférenciées, on entend toute cellule végétale ne présentant aucun des caractères d'une spécialisation particulière et capable de vivre par elle-même et non en dépendance avec d'autres cellules.

Les cellules végétales dédifférenciées peuvent être obtenues à partir de matériel végétal issu de plante entière ou de partie de plante comme les feuilles, les tiges, les fleurs, les pétales, les racines, les fruits, leur peau, l'enveloppe les protégeant, les graines, les anthères, la sève, les épines, les bourgeons, l'écorce, les baies, et des mélanges de ceux-ci.

Préférentiellement, les cellules végétales dédifférenciées sont obtenues à partir d'écorce, de feuilles, de bourgeons et de la peau des fruits, en particulier de la cuticule du fruit.

Les cellules végétales dédifférenciées utilisables selon l'invention peuvent être obtenues à partir de végétaux obtenus par culture in vivo ou issu de culture in vitro.

Par culture in vivo on entend toute culture de type classique c'est à dire en sol à l'air libre ou en serre ou encore hors sol ou en milieu hydroponique.

Par culture in vitro, on entend l'ensemble des techniques connues de l'homme du métier qui permet de manière artificielle l'obtention d'un végétal ou d'une partie d'un végétal. La pression de sélection imposée par les conditions physicochimiques lors de la croissance des cellules végétales in vitro permet d'obtenir un matériel végétal standardisé, exempt de contaminations et disponible tout au long de l'année contrairement aux plantes cultivées in vivo.

Préférentiellement selon l'invention on utilise des cellules végétales dédifférenciées issues de culture in vitro.

Les cellules végétales dédifférenciées utilisables selon l'invention peuvent être obtenues par toute méthode connue de l'art antérieur. A cet égard on peut citer les méthodes décrites par E.F. George et P.D. Sherrington dans Plant Propagation by tissue culture, handbook and directory of commercial laboratories (Exegetics Ltd 1984).

Les milieux de culture utilisables selon l'invention sont ceux généralement connus de l'homme du métier. On peut citer à titre d'exemples les milieux de Gamborg, Murashige et Skoog, Heller, White etc.... On trouvera dans "Plant Culture Média : formulations and uses" de E.F. George, DJM Puttock et H.J George (Exegetics Ltd 1987, tome 1 & 2) des descriptions complètes de ces milieux.

Préférentiellement selon l'invention on prépare les cellules végétales dédifférenciées cultivées sur milieu de Murashige et Skoog.

Etat de la technique

On connaît par le document FR 2795637 une composition cosmétique contenant un extrait de cellules végétales dédifférenciées pour éviter les problèmes d'odeur. Cette composition contient un extrait de cellules végétales dédifférenciées mais non élicitées, de sorte que cette composition est pauvre en métabolites secondaires ou en phytoalexines, voire est sensiblement exempte de tels composés. De plus ce document décrit l'utilisation d'extrait aqueux obtenus après broyage des cellules dans leur milieu de culture puis élimination des particules en suspension avec une perte inévitable des métabolites liés aux particules en suspension. Afin d'éliminer les protéases et notamment les oxydases ce document préconise également l'emploi de filtres capturant les molécules d'un poids moléculaire supérieur à 100.000 daltons perdant ainsi dans l'extrait final tous les métabolites supérieurs à ce poids et qui peuvent s'avérer d'un grand intérêt pour l'industrie cosmétique. De plus afin d'éliminer les problèmes dus à l'oxydation le document préconise l'ajout de stabilisants, notamment la cystéine et/ ou des dérivés soufrés ce qui entraîne nécessairement une pureté moindre de l'extrait avec des étapes subséquentes de filtration. Les méthodes décrites dans ce document nécessitent la mise en oeuvre de moyens compliqués pour l'obtention d'extraits dont, tant la pureté (nombreux additifs), que la qualité et la concentration (en métabolites) n'est pas optimale. De plus les nombreuses étapes nécessaires à l'obtention des extraits de ce procédé induisent des coûts élevés et le risque de contaminations de part les nombreuses manipulations et additifs.

On connaît les cultures de cellules dédifférenciées, d'autre part on connaît les mécanismes d'élicitation de ces cellules suivi d'étapes d'extractions et de filtrations diverses suivis de lyophilisation afin d'incorporer les extraits obtenus dans une préparation cosmétique ou pharmaceutique. De tels procédés sont par exemple décrits dans US 4,241,536 ; EP 378 921, WO 88/00968, EP 1 203 811, etc. pour des espèces de plantes diverses. Le contenu de ces documents est incorporé dans la présente description par référence pour décrire des milieux de culture, des espèces de plantes, des éliciteurs possibles, etc.

Le document EP378921 décrit des méthodes de production de métabolite de plante par élicitation comprenant une étape d'isolation dudit métabolite dudit milieu de production.

Le document FR2808190 décrit des compositions contenant un extrait d'un végétal Vitis vinifera, l'extraction étant opérée au moyen d'un solvant organique.

Le document FR2795637 décrit des compositions comprenant un extrait de cellules végétales dédifférenciées, extrait dont les particules fines ont été éliminées.

L'article "Changes in the phytoalexin content of various Vitis spp. in response to ultraviolet C elicitation", Douillet-Breuil et al, Journal of Agricultural and Food chemistry, US, octobre 1999, volume 47, numéro 10 décrit la production de phytoalexin de Vitis vinifera en fonction d'un rayonnement UV-C.

De nos jours, malgré les compétences et le savoir faire des industries dans le domaine de l'extraction végétale, et malgré les progrès de la chimie organique, plusieurs étapes d'extraction sont indispensables pour obtenir une matière première végétale.

Plusieurs inconvénients sont imputés à ces extractions :

- perte de la structure tertiaire des molécules isolées,
- présence des différents solvants au niveau du produit fini,
- hétérogénéité des substrats nécessitant des extractions fines faisant appel à des solvants de plus en plus toxiques,
- qualité de l'extrait en fonction de l'état physiologique de la plante au moment de la récolte,
- production de l'extrait limitée en fonction des saisons,

Face à ces facteurs limitants et au regain d'intérêt des consommateurs pour tout ce qui est d'origine naturelle, plusieurs tentatives d'obtention de cellules ont été réalisées. Ainsi, à ce jour, deux procédés majeurs ont été exploités :

- La culture de cellules à partir d'organismes unicellulaires ou de microorganismes, technique peu originale se basant sur la reproduction des conditions de vie normale. Cependant ces organismes sont primitifs et ne développent pas de métabolisme secondaire, source des principes actifs les plus intéressants.
- L'obtention des cellules à partir de fruits (cellules fraîches) après digestion enzymatique. Les limites de ce procédé résident dans le fait que les fruits ne sont pas aseptiques et qu'ils peuvent contenir des résidus de pesticides (fongicides, herbicides, insecticides,...). D'autre part, les enzymes (cellulases, pectinases,...) utilisées en quantité importante (2%, P/P) pour la digestion des parois végétales et l'obtention des cellules sans paroi (protoplastes) se retrouvent dans le produit fini. Par ailleurs, les enzymes utilisées peuvent altérer la qualité des métabolites. Enfin, l'utilisation de cette technique permet seulement de récupérer des protoplastes (cellules sans paroi cellulaire), structures fragiles ne pouvant pas orienter leur métabolisme.

Les inventeurs ont développé une technologie innovante et maîtrisée, garantissant la qualité et l'authenticité des produits. Il s'agit de la mise en culture de cellules de plantes supérieures dédifférenciées.
En effet, et pour la première fois, un procédé industriel permet d'obtenir des cellules à partir de plantes supérieures par un processus sans aucune modification de leur patrimoine génétique, permettant à la cellule de garder ses caractéristiques physiologiques.

Le maintien des différentes souches est rassuré par repiquages réguliers, avec une maîtrise totale des différentes conditions de culture.
L'intérêt de ce procédé et qu'il permet la culture de cellules végétales dédifférenciées en grands volumes tout en répondant aux besoins de l'industrie, notamment :

- Le respect de la structure tertiaire des molécules,
- L'absence de solvant et de résidus,
- L'homogénéité des substrats,
- La production continue indépendamment du cycle des saisons,
- La conservation des caractéristiques biologiques et physiologiques sans ajout de conservateur,
- L'absence totale de polluants,
- La production standardisée et reproductible quant à la qualité et la concentration des métabolites,
- L'utilisation de ces suspensions végétales après lyophilisation directe avec utilisation de température inférieure à -30°C. Cette technique permet l'obtention d'une poudre très fine pouvant être dispersée dans des compositions cosmétiques (crèmes, pommades, lotions...). Ces cellules sont susceptibles de libérer directement les principes actifs qu'elles contiennent, sans passage par une extraction à l'aide de solvants organiques (élimination des risques de résidus). Toutefois, il est préférable de soumettre le produit de lyophilisation à un broyage pour éviter toute agglomération de particules.
- L'utilisation des extraits cellulaires uniquement après sonification et centrifugation.

Cette technologie apporte une alternative utile et innovante aux extractions conventionnelles par solvants. La possibilité d'orienter de manière naturelle (élicitation) la synthèse des métabolites sans porter atteinte à l'intégrité génétique des cellules, représente une garantie de qualité et d'authenticité.

De manière tout à fait surprenante les inventeurs ont découvert que l'on pouvait directement incorporer ou disperser les cellules après élicitation, séchage et broyage dans une composition cosmétique et/ ou pharmaceutique. La composition selon l'invention contient alors des membranes de cellules, des organites cytoplasmiques et de la matière vacuolaire. Ce procédé présente notamment l'avantage de désactiver les enzymes oxydants sans ajouts d'additifs ou de produits chimiques. Un autre aspect de l'invention permet de concentrer et de diriger la production de phytoalexines sans pertes quantitatives ou qualitatives dues aux extractions et aux filtrages. Un aspect particulier de l'invention est qu'il évite les étapes d'extraction et de filtrage et permet l'obtention d'un broyat de cellules dénué d'additifs, de solvants et de résidus, ledit broyat pouvant être directement dispersé dans une composition cosmétique. La composition selon l'invention contient au moins un broyat de cellules végétales dédifférenciées et élicitées en culture in vitro pour synthétiser au moins une phytoalexine, cette élicitation pour synthétiser au moins une phytoalexine étant avantageusement opérée après une étape de culture in vitro de cellules végétales sans élicitation, dans laquelle ledit broyat contenant au moins une phytoalexine comprend au moins 95%, avantageusement au moins 97%, de préférence au moins 99% en poids de l'ensemble des matières sèches issues des cellules végétales broyées dédifférenciées et élicitées in vitro, ledit broyat étant dispersé dans ladite composition ou étant sous une forme apte à être dispersée dans ladite composition. En particulier, le broyat contient sensiblement toutes les matières sèches (après extraction de l'eau présente dans les cellules) des cellules végétales dédifférentiées et élicitées broyées. Un tel broyat riche en phytoalexine par rapport au contenu des cellules non élicitées contient toutefois toutes les matières naturelles présentes dans les cellules.

Le but de la présente invention est entre autre de proposer un procédé simple excluant l'emploi d'additifs et de produits chimiques et respectant le caractère naturel des cellules obtenues. De plus, l'élicitation par des moyens physiques permet de diriger et de concentrer la production de métabolites recherchés par l'industrie cosmétique. L'aspect particulier de l'invention en respectant l'intégrité des cellules obtenues et en les élicitant permet d'une part d'optimiser la concentration et la qualité des métabolites obtenus, d'autre part de résoudre les problèmes d'oxydation en inactivant les enzymes par un simple séchage (lyophilisation) sans ajout d'additifs et sans pertes dues aux filtrations et enfin permet la dispersion du broyat obtenu directement dans une préparation cosmétique.

Par élicitation dans le milieu de culture, on entend la mise en oeuvre d'un stress ou d'une agression (biologique, chimique ou physique) sur les cellules dans leur milieu de culture afin de provoquer un ou plusieurs mécanismes de défense.

Tout au long de leur développement, les plantes sont soumises à des agressions continuelles de la part de leur environnement. Cependant, elles se montrent souvent capables de résister naturellement à ces agressions extérieures grâce à la présence ou à l'activation de mécanismes de défense (Hammond-Kosack et Jones, 1996). Ainsi, bien que certains de ces mécanismes soient constitutifs et fournissent une barrière physique et chimique à une agression, d'autres ne sont induits qu'après une attaque par un nuisible.

Dès qu'une plante détecte un pathogène, elle met en place un des systèmes de défense naturelle les plus efficaces : la réponse d'hypersensibilité. Au niveau du site de pénétration du pathogène, cette réaction, rapide et violente, se traduit par la mort des premières cellules infectées et l'apparition d'une petite zone nécrotique, isolant ainsi les cellules attaquées du reste de la plante (Dang1 et al., 1996 ; Lamb et Dixon, 1997). Le déclenchement de cette réponse dépend d'une reconnaissance spécifique de l'agent pathogène par la plante hôte. En effet, la plante attaquée reconnaît par l'intermédiaire d'une protéine (récepteur) une protéine produite par le pathogène, nommée éliciteur. Chez la plante, les gènes codant pour les protéines réceptrices sont appelés gènes de résistance (gènes R), et pour le pathogène, les gènes codant pour les molécules élicitrices sont nommés gènes d'avirulence (gène Avr) : on parle alors ici d'une relation gène pour gène ou relation R-Avr (Hammond-Kosack, 1996). D'autres molécules, qualifiées d'éliciteurs généraux, sont également capables d'initier (de façon moins spécifique que les éliciteurs cités précédemment) cette réaction de défense de la plante hôte. Celles-ci, sont le plus souvent des oligosaccharides libérés par le pathogène (éliciteurs exogènes) ou la cellule végétale (éliciteurs endogènes), (Scheel et Parker, 1996).

A cette réaction d'hypersensibilité succède l'activation d'une réaction de défense intense dans les cellules voisines de la zone infectée, c'est la réaction locale. Enfin, l'émission de divers signaux d'alerte vers toutes les autres organes de la plante, ce qui lui permet de réagir plus rapidement et plus efficacement lors d'une nouvelle agression, on parle alors de réaction systémique.

Lors de ces réactions, trois catégories de systèmes de défense peuvent être activés :

- la formation d'un épiderme de cicatrisation et le renforcement des parois (lignification...) (Dai et al., 1995) ;

- la synthèse de protéines de défense ou «Pathogenesis Related» (PRs) proteins découvertes en 1970 chez le Tabac. Parmi ces PRs on trouve, par exemple, des inhibiteurs de protéase (Ryan, 1992), des enzymes hydrolytiques, comme les chitinases ou les ß-1,3 glucanases (Derckel et al., 1996 ; Robinson et al., 1997, Kraeva et al., 1998 ; Salzman et al., 1998 ; Renault et al., 2000) ;

- et la synthèse de métabolites secondaires de type phytoalexines. Parmi, les métabolites secondaires, plus de 300

phytoalexines ont déjà été caractérisés. Elles appartiennent à un large spectre de classes chimiques différentes parmi lesquelles on pourra citer les coumarines, les benzofuranes, les terpènes, les alcaloïdes, certains polyphénols (Smith,1996)...

La mise en place des réactions de défense des plantes implique toute une panoplie de signaux de transduction conduisant à l'induction rapide de l'expression de gènes de défense. Ainsi, la reconnaissance du pathogène par la plante hôte va activer toute une cascade de signaux dans les cellules attaquées tels que la phosphorylation de protéines par des protéines kinases, les flux d'espèces ioniques ($Ca^{2+}$), la formation d'espèces oxygénées réactives (Coté et Hahn, 1994 ; Shibuya et al., 1996 ; Benhamou, 1996)...

De plus, les cellules attaquées sont capables de produire des signaux d'alarme transmis au cellules voisines (réaction locale) ainsi qu'à toute la plante engendrant ainsi, comme énoncé dans le paragraphe précédent, le phénomène de réaction systémique.

Le mécanisme de résistance systémique le plus étudié est le phénomène de SAR ou «systemic acquired resistance». Le terme de SAR a été défini par Ross en 1961. Il décrit l'apparition de résistance d'une plante consécutive à une attaque par un pathogène, aussi bien dans les parties infectées que dans les parties saines de la plante. Elle se développe, en général, après l'apparition de lésions nécrotiques autour du site d'inoculation. Cette réponse d'hypersensibilité localisée restreint le pathogène dans, et autour du site d'infection, et semble rendre la plante plus résistante aux agressions par divers organismes (Ryals et al., 1996). Comment les parties distantes du site d'infection sont-elles capables d'acquérir cette résistance? C'est en 1966 que Ross a développé l'idée de l'existence de molécules signal qui, à de faibles concentrations, seraient capables d'activer des mécanismes de défense dans les tissus distants de la zone infectée.

Trois types de molécules peuvent intervenir chez les plantes comme signal d'alarme au niveaux intra et intercellulaire, à courte ou longue distance : l'acide salicylique, l'éthylène et les jasmonates.

Le mécanisme de transduction le plus étudié et le mieux connu dans le développement de la SAR est celui impliquant l'acide salicylique (AS) (Delaney et al., 1994 ; Ryals et al., 1996).

D'une part, l'acquisition de résistance de certaines plantes est directement liée à une augmentation de la synthèse endogène d'AS ou de ses dérivés (Malamy et al., 1990 ; Métraux et al., 1990 ; Rasmussen et al., 1991 ; Smith-Becker et al., 1998). Dans ce type de réponse, lorsque la synthèse d'AS est initiée, la transduction du signal s'effectuerait à longue distance, soit par transport direct de l'AS (Shulaev et al., 1995), soit par conversion de ce dernier en méthyl salicylate (MeSa), molécule-signal volatile pouvant entraîner l'apparition de résistance dans les tissus sains de la plante infectée mais aussi dans les plantes voisines (Shulaev et al., 1997). De plus, la mise en évidence du rôle joué par l'AS dans le déclenchement de la SAR est liée à l'utilisation de plants de Tabac transgéniques exprimant le gène bactérien NahG. Ce gène, codant pour la salicylate hydroxylase, inactive l'AS en le convertissant en catéchol, rendant ainsi les plantes transformées incapables de développer une SAR (Gaffney et al., 1993).

D'autre part, certaines études montrent que l'application d'AS exogène est capable d'induire une résistance à différentes lésions causées par une bactérie, un virus ou un pathogène. Ainsi, chez le Tabac, on observe une réduction des symptômes liés à l'inoculation du virus de la Mosaïque du Tabac après traitement des plants par l'AS (White et al., 1983). De même, l'AS est capable de stimuler la biosynthèse de différentes protéines PRs normalement produites dans le cas d'une SAR (Renault et al., 1996 ; Narusaka et al., 1999).

Différentes études ont montré que certaines réactions de défense pouvaient être activées indépendamment de la présence d'AS. Ainsi, d'autres types de molécules ont été reconnues comme molécules-signal de la SAR (Enyedi et al., 1992 ; Pieterse et al., 1999). Ces réactions feraient intervenir principalement deux régulateurs de croissance végétale, l'éthylène et l'acide jasmonique (AJ), comme signaux d'induction de défense systémique. En effet, ces deux types de molécules peuvent être rapidement produites, de façon endogène, lorsqu'une plante est soumise à une agression, et entraîner ainsi l'apparition d'une résistance. De, plus elles sont également capables d'induire l'expression de certaines PRs et/ou la production de phytoalexines.

L'éthylène est une phytohormone volatile intervenant dans de nombreux processus physiologiques des plantes. Son rôle dans les phénomènes de défense végétale a été démontré par plusieurs équipes. Des études ont montré que l'attaque d'une plante par un pathogène ou un herbivore pouvait être corrélée à une induction ou une augmentation de la synthèse d'éthylène endogène dans la plante hôte (O'Donnell et al., 1996 ; Popp et al., 1997 ; Lund et al., 1998). De plus, tout comme l'AS, l'application d'éthylène exogène est également capable de stimuler la synthèse de protéines PRs (Ward et al., 1991 ; Penninckx et al., 1996 ; Yang et al., 1997). Enfin, l'activation de protéines de défense chez Arabidopsis en réponse à un pathogène peut être bloquée dans le cas de mutants déficients dans la perception de ce signal (Pennickx et al., 1998).

L'acide jasmonique et son méthyl ester, le méthyl jasmonate (MeJa), sont des composés naturels de type cyclopentanone analogues aux prostaglandines animales par leur biosynthèse et leur fonction (Creelman et al., 1992 ; Mason et al., 1993 ; Sadka et al., 1994). Ils dérivent des acides gras et sont synthétisés à partir de l'oxydation lypoxygénase-dépendante de l'acide $\alpha$-linolénique. On parle de voie octadécanoïque.

Outre leur rôle dans diverses fonctions physiologiques (Stawick, 1992), ces composés ont récemment été impliqués en tant que molécules de signalisation intracellulaire synthétisées en réponse à un stress, biotique ou abiotique, et menant aux réponses de défense des plantes, comme la biosynthèse de phytoalexines ou de protéines de défense (Gundlach et al., 1992 ; Blechert et al., 1995 ; Creelman et al., 1995 ; Doares et al., 1995 ; Conconi et al., 1996a et 1996b ; Ignatov et al., 1996 ; Baldwin et al., 1997).

D'une part, l'application de MeJa, dérivé volatile de l'acide jasmonique, induit la production de nombreux métabolites secondaires, in vivo ou in vitro (en cultures cellulaires), dont certains jouent le rôle de composés de défense. En effet, cet ester est capable d'éliciter la biosynthèse de furanocoumarines dans les feuilles de Céleri (Miksch et Boland, 1996), de momilactone A dans des cultures cellulaires de Riz (Nojiri et al., 1996), d'alcaloïdes de jeunes plants ou de cultures cellulaires de Catharanthus roseus (Aerts et al., 1996 ; Gantet et al., 1997) et de jeunes plants de Cinchona ledgeriana (Aerts et al., 1994 ; 1996). D'autres dérivés précurseurs de l'AJ, comme l'acide 12-oxo-phytodiénoïque, sont capables d'agir sur la biosynthèse de nombreux métabolites secondaires.

L'effet inducteur de ces composés est souvent précédé d'une activation de l'expression des gènes ou de la synthèse d'enzymes intervenant dans la biosynthèse de ces divers métabolites. Parmi ces enzymes, on trouve la phénylalanine ammonialyase (PAL), la 4-coumarate-CoA ligase (4CL), la chalcone synthase (CHS), la dihydroflavonol-4-reductase (DFR), la polyphénol oxydase (PPo), la bercaptol methyl transférase (BMT), la tyrosine/dopa décarboxylase (TYDC), ... (Dittrich et al., 1992 ; Gundlach et al., 1992 ; Mizukami et al., 1993 ; Tamari et al., 1995 ; Ellard-Ivey et al., 1996 ; Facchini et al., 1996 ; Ignatov et al., 1996 ; Lee et al., 1997 ; Yasaki et al., 1997 ; Constanbel et Ryan, 1998).

D'autre part, l'exposition de plantes à des vapeurs de MeJa, permet d'éliciter des mécanismes de défense similaires à ceux induits par des insectes, des herbivores, une blessure ou les UV (Farmer et Ryan, 1990 ; 1992 ; Conconi et al., 1996b; Ozawa et al., 2000). Ainsi, les jasmonates, et en particulier le MeJa, sont capables d'induire la biosynthèse de protéines de défense et de stress, encore appelées JIPs (Jasmonate Induced Proteins), (Reinbothe et al., 1994).

De plus, le traitement de plants de Tomate, de Pomme de terre ou de Luzerne par le MeJa, induit l'expression d'inhibiteurs de protéase (Farmer et Ryan, 1990, 1992 ; Hildmann et al., 1992 ; Pena-Cortes et al., 1992 ; Lee et al., 1996), la biosynthèse de thionine chez l'Orge (Reinbothe et al., 1997), de protéines riche en proline ou de Vsp (Vegetative storage protéins) des graines de Soja (Stawick et al., 1991 ; Creelman et al., 1992 ; Mason et al., 1992, 1993 ; Berger et al., 1995).

Enfin, il est également impliqué dans la régulation de la biosynthèse de protéines participant à la transduction des signaux en réponse à un stress, comme par exemple les lypoxygenases (Saravitz et Siedow, 1996) ou bien encore la systémine (Reinbothe et al., 1994 ; Bergey et al., 1996).

De ce fait, il n'est pas étonnant que différentes équipes aient montré une diminution de l'incidence de certaines maladies suite à un traitement de la plante concernée par les vapeurs de MeJa (Cohen et al., 1993 ; Meir et al., 1998 ; Thomma et al., 1998 ; Vijayan et al., 1998 ; Thomma et al., 2000). De plus, J.S. Thaler (1999) montre que pour de nombreuses plantes, divers mécanismes de défense contre l'attaque d'herbivores sont induits par l'intermédiaire de la voie octadécanoïque et seraient ainsi impliqués dans l'attraction d'ennemis naturels. Par exemple, elle démontre que le traitement de plants par l'AJ augmente le parasitisme des chenilles de nuisibles.

Chez la Vigne, les mécanismes de signalisation impliqués dans l'expression des réactions de défense ne sont pas encore bien connus. Cependant, on retrouve la synthèse des trois types de molécules de défense (lignine, protéines de défense et phytoalexines). En particulier, le rôle de phytoalexines est notamment tenu par une famille de composés originaux : les polyphénols (Deloire et al., 2000).

Présents en plus ou moins grandes quantités dans tous les organes de la plante, les phytoalexines ils sont inductibles au niveau des feuilles et des baies. Ce type d'induction est désigné sous le terme d'élicitation. Les facteurs d'élicitation (ou éliciteurs) peuvent avoir des origines différentes. Il peut s'agir :

- d'élicitation biotique, par exemple lors d'une agression par un pathogène comme Botrytis cinerea, agent de la Pourriture grise (Jeandet et al., 1995 ; Bavaresco et al., 1997), Plasmopara viticola, agent du Mildiou (Dercks et Creasy, 1989) ou Phomopsis viticola, responsable de l'Excoriose (Hoos et Blaich, 1990).

- d'élicitation abiotique par les facteurs environnementaux tels que U.V., température, lumière, asphyxie, agents naturels extraits d'autres plantes (Jeandet et al., 1997 ; Langcake et Pryce, 1977b ; Douillet-Breuil et al., 1999), le chlorure d'aluminium (Adrian et al., 1996) ou l'ozone (Sarig et al., 1996).

Lors d'une élicitation, des phytoalexines comme le trans-resvératrol, le trans-picéide, l'ε-viniférine et le ptérostilbène, peuvent être induits au niveau des feuilles et des baies (Soleas et al., 1997). Cette propriété de biosynthèse de novo des phytoalexines en réponse à un stress, et en particulier après attaque par un pathogène, suggère que ces molécules pourraient jouer le rôle de moyen de défense naturelle des plantes.

Ce rôle de molécules de défense est corroboré par certaines études qui semblent indiquer une corrélation

étroite entre le niveau de résistance naturelle de la plante et son aptitude à synthétiser ces molécules. Par exemple, Langcake et Mc Carthy (1979) ont mis en évidence une relation entre la résistance de certaines espèces du genre Vitis à Botrytis cinerea ou Plasmopara viticola et leur capacité de biosynthèse de phytoalexines (resvératrol et viniférine). De plus, Dercks et Creasy (1989) ont montré que les espèces résistantes à Plasmopara viticola produisent cinq fois plus de phytoalexines que les espèces sensibles. De même, à l'intérieur de l'espèce Vinifera, on trouve des cépages plus ou moins tolérants à l'attaque par des champignons selon leur capacité de production de phytoalexines.

L'élicitation des cellules peut être opérée au moyen d'agents ou de stress divers, tels que pression, dépression, vide, variation de pression, présence d'un gaz, atmosphère variable, température, froid, lumière, cycle de luminosité, radiation, toxine, toxine végétale, agitation, bactérie, virus, fungi, microorganisme, ultrason, IR, UV, asphyxie, etc.

Toute méthode d'élicitation connue de l'homme du métier peut être utilisée pour préparer un broyat utilisable dans la composition selon l'invention.

Ainsi, les broyats utilisables selon l'invention peuvent prendre toute forme connue. On peut, en particulier, citer les broyats aqueux, alcooliques, notamment éthanoliques ou encore hydroalcooliques.

Préférentiellement selon l'invention, le broyat est un broyat aqueux ou un broyat sec ou sensiblement sec.

Avantageusement, le broyat peut dans une étape subséquente être lyophilisé. Selon une forme avantageuse, le broyat est un broyat des cellules dans leur milieu de culture ou dans un extrait de milieu de culture, ledit broyat étant par après avantageusement séché ou lyophilisé.

L'invention a donc pour objet une composition cosmétique contenant au moins un milieu de cellules végétales dédifférenciées, caractérisée en ce que ledit milieu est un broyat, de cellules végétales dédifférenciées, cultivées dans un milieu de culture in vitro et élicitées dans le milieu de culture in vitro, d'au moins une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces, ledit broyat étant dispersé dans ladite composition ou étant sous une forme apte à être dispersé dans ladite composition. Les cellules végétales sont cultivées dans un milieu de culture in vitro et sont élicitées dans un milieu de culture in vitro pour synthétiser au moins une phytoalexine, cette élicitation étant avantageusement opérée après une étape de culture in vitro des cellules végétales sans ou sensiblement sans élicitation pour synthétiser au moins une phytoalexine. Ledit broyat contient au moins une phytoalexine comprend au moins 95%, avantageusement au moins 97%, de préférence au moins 99% en poids de l'ensemble des matières sèches issues des cellules végétales broyées dédifférenciées et élicitées in vitro, ledit broyat étant dispersé dans ladite composition ou étant sous une forme apte à être dispersée dans ladite composition. De manière préférée, sensiblement tout, voire tout l'ensemble des matières sèches issues des cellules végétales broyées dédifférenciées et élicitées in vitro.

Au cas où les cellules sont lavées pour éliminées tout milieu de culture présent, sans affecter la structure membranaire des cellules, il est possible d'obtenir un broyat de cellules dédifférenciées élicitées ne contenant pas de milieu de culture (par exemple moins de 0,1 % en poids par rapport au poids des cellules broyées). Avantageusement, le broyat de cellules végétales dédifférenciées et élicitées in vitro comprend des particules provenant des vacuoles, des particules provenant du cytoplasme et des particules provenant de la membrane pecto cellulosique, ledit broyat contenant au moins 0,1 % en poids de phytoalexine(s).

Par exemple, la composition contient de 0,005 à 25% en poids, avantageusement de 0,005 à 5 % en poids de milieu ou broyat de cellules végétales dédifférenciées, ce poids étant calculé sous forme sèche.

De façon avantageuse, la composition contient un milieu ou broyat de cellules végétales dédifférenciées et élicitées in vitro. De préférence, le broyat est un broyat sec ou sensiblement sec de cellules végétales dédifférenciées et élicitées en milieu de culture in vitro, ledit broyat sec ou sensiblement sec contenant une teneur en eau inférieure à 25% en poids, avantageusement inférieure à 15% en poids, de préférence inférieure à 10% en poids. En particulier, ledit broyat sec ou sensiblement sec contient une quantité d'eau suffisante pour assurer l'intégrité de la membrane des cellules avant l'étape de broyage.

Le broyat a avantageusement une granulométrie moyenne de particules solides inférieure à 100 $\mu$m, avantageusement inférieure à 10 $\mu$m, de préférence inférieure à 1 $\mu$m. Avantageusement, les particules du broyat ont une granulométrie telle que 90% en poids des particules de cellules broyées ont une granulométrie comprise dans la plage granulométrie moyenne - 25% jusque granulométrie moyenne + 25%.

Selon une forme de réalisation particulière, ledit broyat de cellules végétales dédifférenciées et élicitées en milieu de culture in vitro contient au moins une phytoalexine synthétisée par l'élicitation des cellules végétales dédifférenciées en milieu de culture in vitro, ou un mélange de telles phytoalexines.

Selon une particularité avantageuse d'une forme de réalisation, ledit broyat de cellules végétales dédifférenciées et élicitées est un broyat de cellules végétales dédifférenciées et élicitées par un agent dans le milieu de culture in vitro, ledit broyat étant sensiblement exempt dudit agent après élicitation en milieu de culture in vitro.

Selon une autre particularité avantageuse d'une forme de réalisation, ledit broyat de cellules végétales dédifférenciées et élicitées est un broyat de cellules végétales dédifférenciées et élicitées par un agent dans le milieu de culture, ledit agent étant un agent dont la présence est souhaitée dans la composition cosmétique, tel que par exemple, un tensioactif, un agent dispersant, un acide, etc.

Selon une forme de réalisation préférée, les cellules dédifférenciées sont élicitées en milieu de culture in vitro par un agent volatile, en particulier un gaz, tel que du $CO_2$.

Selon une forme de réalisation préférée, les cellules dédifférenciées sont élicitées en milieu de culture in vitro par un agent physique, en particulier la température, la lumière, les champs électromagnétiques, les UV, la pression, l'asphyxie.

Selon un détail d'une forme de réalisation, ledit broyat de cellules végétales dédifférenciées et élicitées en milieu de culture in vitro contient au moins un composé terpénique ou tannique ou polyphénolique, ledit composé étant synthétisé par l'élicitation in vitro des cellules végétales dédifférenciées dans leur milieu de culture.

Avantageusement, le broyat de cellules végétales dédifférenciées et élicitées en milieu de culture in vitro se présente sous la forme d'une suspension visqueuse ou d'un gel ou d'un broyat sensiblement sec, ladite suspension, gel ou broyat étant sous une forme dispersible dans la composition.

Selon une forme de réalisation particulière, le broyat de cellules est un broyat de cellules de vigne dédifférenciées et élicitées en milieu de culture in vitro.

Selon un détail d'une forme de réalisation préférentielle, la composition contient un broyat de cellules dédifférenciées, cultivées et élicitées dans leur milieu de culture in vitro.

Selon une forme de réalisation avantageuse, la composition contient un milieu de cellules dédifférenciées et élicitées en milieu de culture in vitro, en particulier un broyat de cellules dédifférenciées et élicitées, ledit milieu ou broyat contenant au moins 0,1 % en poids de stilbènes par rapport au poids sec des cellules, en particulier au moins 0,2 % en poids de stilbènes par rapport au poids sec des cellules, de préférence au moins 0,5 % en poids de stilbènes par rapport au poids sec des cellules.

Dans la composition suivant l'invention, le broyat est issu de la culture de cellules végétales dédifférenciées, élicitées en milieu de culture in vitro puis séchées des espèces Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, Cacao, psoralea coryilfolia, vitex negundo, commiphora wighii,, eucalyptus punctata, lavandula angustifolia, citrus limon, vanilla planifolia, marrubium vulgare, pilocarpus jaborandi, roses, betula, thé, et les mélanges de cellules de telles espèces.

Selon une forme de réalisation possible, la composition suivant l'invention comprend des cellules végétales dédifférenciées élicitées broyées (par exemple avec une granulométrie inférieure à 5$\mu$m) et des cellules végétales dédifférenciées élicitées non broyées ou broyées plus grossièrement, les cellules végétales dédifférenciées élicitées non broyées ou broyées plus grossièrement étant identiques ou différentes aux cellules élicitées broyées et/ou présentant une élicitation différente des cellules élicitées broyées. Selon une forme de réalisation particulière, les cellules végétales dédifférenciées élicitées sont séparées en deux fractions distinctes, une première étant soumise à un broyage fin, tandis que la seconde est soumise à un broyage grossier ou n'est pas broyée.

L'invention a encore pour objet, un procédé de préparation d'une composition à usage topique selon l'invention. Dans ce procédé, on met des cellules végétales dédifférenciées d'au moins une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces dans un milieu de culture in vitro de manière à permettre une croissance des cellules, on élicite lesdites cellules végétales différenciées dans leur milieu de culture in vitro pendant une période de temps suffisante pour la synthèse d'une quantité suffisante de métabolite(s), en particulier d'au moins un métabolite secondaire, de préférence d'au moins une phytoalexine ou d'un mélange de phytoalexine, et on mélange au moins un milieu ou broyat de cellules végétales élicitées du milieu de culture, avec un ou plusieurs excipients pour préparer une composition à usage topique, en particulier cosmétique, par exemple pour le traitement de la peau, des cheveux, du cuir, des ongles, etc.. Les cellules élicitées sont broyées avant et/ou après leur mélange avec un ou plusieurs excipients de la composition. Bien qu'il soit possible de soumettre les cellules dédifférenciées et élicitées en milieu de culture in vitro à un broyage, il est avantageux de séparer les cellules dédifférenciées et élicitées du milieu de culture en n'affectant sensiblement pas les membranes des cellules et de soumettre lesdites cellules ensuite à un broyage, avantageusement après une ou des étapes de lavage n'affectant sensiblement pas les membranes des cellules et/ou après une ou des étapes de séchage.

Selon une forme de réalisation particulière, les cellules végétales dédifférenciées sont soumises successivement à des étapes de culture in vitro sans élicitation et à des étapes de culture in vitro avec élicitation.

De façon avantageuse, on sépare des cellules élicitées du milieu de culture in vitro, et on soumet ledit extrait à un séchage, suivi d'un broyage.

Bien qu'il soit possible de lyophiliser les cellules dédifférentiées élicitées séparées et lavées sans affecter la structure des membranes des cellules et de broyer ensuite le lyophilisat, il est avantageux de soumettre les cellules dédifférentiées élicitées séparées et lavées sans affecter la structure des membranes des cellules, à un séchage contrôlé de manière à ne pas affecter sensiblement la barrière membranaire (température de séchage de moins de 50°C, teneur en eau des cellules d'au moins 3%, par exemple de 5 à 15%), et ensuite à un broyage. Ceci permet de casser les cellules (membranes, cytoplasme et vacuoles) lors de l'étape de broyage, de manière à assurer la libération des phytoalexines lors de cette étape.
L'étape de broyage et/ou l'étape de séchage (en particulier contrôlée) et/ou l'étape de lavage et/ou l'étape de séparation des cellules dédifférentiées sont avantageusement réalisée en présence d'un ou de plusieurs agents antioxydants, tels que vitamine E, etc., pour éviter ou réduire toute oxydation d'un ou de plusieurs composés de la cellules (par exemple de la membrane).

De préférence, on élicite les cellules dans leur milieu de culture in vitro au moyen d'un agent, qui après extraction des cellules élicitées ne se retrouve pas dans le broyat de cellules élicitées.

Avantageusement, dans le procédé suivant l'invention, on contrôle l'étape d'élicitation desdites cellules végétales différenciées dans leur milieu de culture in vitro, de manière à obtenir un milieu contenant au moins 0,1 % en poids de stilbènes par rapport au poids sec des cellules dédiférentiées, en particulier au moins 0,2 % en poids de stilbènes par rapport au poids sec des cellules, de préférence au moins 0,5 % en poids de stilbènes par rapport au poids sec des cellules.

Le milieu ou broyat est issu de la culture de cellules végétales dédifférenciées, élicitées en milieu de culture in vitro, puis avantageusement séchées, des espèces Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, Cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé, et les mélanges de cellules de telles espèces.

Un exemple de préparation de broyat utilisable selon l'invention est donné par ailleurs dans les exemples.

La quantité de broyat présent dans la composition selon l'invention est bien entendu fonction de l'effet recherché et peut donc varier dans une large mesure. Pour donner un ordre de grandeur, on peut utiliser un broyat tel que défini précédemment en une quantité représentant de 0,0 1 % à 20% du poids total de la composition et préférentiellement en une quantité représentant de 0,1% à 5% du poids total de la composition.

Le broyat de cellules végétales dédifférenciées et élicitées en milieu de culture in vitro, en particulier pour favoriser la production d'une ou de plusieurs phytoalexines, est par exemple utilisé en tant qu'agent antioxydant, agent anti-radicalaire, agent apaisant, agent anti-irritant, agent scavenger de radicaux libres.

Dans un aspect particulier le procédé de l'invention permet l'obtention d'un broyat enrichi en flavonoïdes tels les flavanols, les anthocyanes et les flavonols.

Dans un autre aspect particulier le procédé de l'invention permet l'obtention d'un broyat enrichi en composés non flavonoïdes tels les acides phénols, dérivés de l'acide benzoïque mais également des composés originaux comme les stilbènes, les isomères trans- et cis- du resvératrol et leurs glucosides, les trans- et cis-picéides.

Dans un autre aspect particulier de l'invention le broyat enrichi en polyphénols, phytoalexines et métabolites secondaires retiendrait leurs effets pharmacologiques importants. Ainsi, les broyats de l'invention présentent une ou plusieurs activités choisies parmi antioxydant, anti-radicalaire, antiinflammatoire, anti-proliférative, relaxante, vasculaire,...etc.

Contrairement aux métabolites primaires, les métabolites secondaires tels que les polyphénols s'accumulent dans la plante in vivo en faible quantité. Dans un aspect de l'invention afin d'obtenir ces métabolites secondaires en quantité importante et de façon continue et stable, nous avons stimulé leur biosynthèse dans des cultures de cellules standardisées.

Particulièrement selon l'invention on utilise des cellules végétales dédifférenciées provenant de végétaux des genres séquoia, vigne, arganier, cacao et chlorophytum, et des combinaisons de celles-ci.

Bien entendu les broyats de cellules végétales dédifférenciées utilisables dans la composition selon l'invention peuvent provenir de mélanges de cellules végétales dédifférenciées obtenues à partir de genres végétaux différents et/ou obtenues à partir de matériel végétal différent, lesdites cellules étant élicitées dans un même milieu de culture in vitro ou dans des milieux de culture in vitro différents (par exemple pour opérer des élicitations différentes) .

Par composition à usage topique on entend des crèmes, des onguents, des lotions, suspensions, des bâtons, des shampooings, des gels, des solutions (par exemple applicable par spray). La composition à usage topique est par exemple une composition cosmétique, dermatologique, une composition d'hygiène de la peau, un parfum, etc.

Préférentiellement selon l'invention, la composition est une composition cosmétique, particulièrement d'application topique.

La présente invention a en outre pour objet un procédé de traitement cosmétique de la peau, caractérisé par le fait que l'on applique sur la peau, sur les cheveux, et/ ou sur les muqueuses, une composition selon l'invention comprenant au moins un broyat de cellules végétales dédifférenciées et élicitées en milieu de culture in vitro, lyophilisées et incorporées ou dispersées dans un produit cosmétique.

Le procédé de traitement cosmétique de l'invention peut être mis en oeuvre notamment en appliquant les compositions cosmétiques telles que définies ci-dessus, selon la technique d'utilisation habituelle de ces compositions. Par exemple: application de crèmes, de gels, de sérums, de lotions, de laits, de shampooings ou de compositions antisolaires sur la peau.

Les caractéristiques essentielles de compositions suivant l'invention, de procédés suivant l'invention, et de broyats suivant l'invention sont données dans les revendications. Les exemples et compositions suivants illustrent l'invention sans la limiter aucunement. Dans les compositions les proportions indiquées sont des pourcentages en poids. Dans ces exemples, on a utilisé un procédé préféré tel que défini en résumé ci-après.

<u>Procédé d'obteption d'un broyat</u>

Etape 1 : Préparation de cellules dédifférenciées et cultivés dans un milieu de culture in vitro

Etape 2 : Elicitation des cellules dédifférenciées dans le milieu de culture

Etape 3 : Extraction des cellules dédifférenciées et élicitées en milieu de culture in vitro, par exemple par filtration du milieu de culture suivi d'une ou de plusieurs étapes de lavage, en particulier opérées pour ne pas détruire la structure des membranes des cellules. Les cellules sont avantageusement lavées pour éliminer sensiblement toute trace de milieu d culture, ce lavage étant opéré de manière à ne pas détruire la structure de la membrane des cellules.

Etape 4 : Séchage ou lyophilisation des cellules dédifférenciées et élicitées en milieu de culture in vitro, cette opération de séchage étant avantageusement opérée pour ne pas détruire la structure des membranes des cellules. Cette étape est avantageusement opérée à une température inférieure à 60° C, par exemple entre -60° C et 50°C.

Etape 5 : broyage (l'étape 5 est avantageusement opérée, toutefois dans certains cas, cette étape peut s'avérer non nécessaire). Le broyat contient ainsi sensiblement tous les composants secs formant la cellule, à savoir sensiblement tous les composant de la membrane, du cytoplasme et des vacuoles.

Etape 6 : mélange et / ou incorporation à un ou des excipients et/ ou à d'autres principes actifs (notamment d'autres cellules/ broyats végétaux) pour la préparation de la composition à usage topique

Exemple 1 <u>Réalisation d'un broyat de cellules dédifférenciées et élicitées in vitro de vigne.</u>

La première étape pour le développement de cultures cellulaires végétales consiste à sélectionner la plante produisant les substances recherchées. On admet aujourd'hui qu'au sein d'une même espèce, il existe une variabilité des capacités de production d'un métabolite donné, en partie d'origine génétique. Lorsque cela est possible, il faudra donc exploiter cette variabilité en sélectionnant le meilleur génotype, c'est-à-dire le plus productif pour le métabolite recherché. A partir de fragments aseptisés d'un organe de plante choisie (feuille, tige, racine...), placés in vitro sur un milieu solide (gélosé), on parvient à induire des proliférations primaires. Ainsi, après quelques semaines de mise en culture, il se forme, au niveau des explants, des amas cellulaires indifférenciés, appelés cals. La croissance de ces cals sera maintenue par repiquages successifs sur un milieu nutritif neuf. Les conditions de culture vont alors induire l'apparition spontanée d'une variabilité morphologique et métabolique entre cals issus d'une même plante ou d'un même explant. Cependant, le maintien de conditions environnementales constantes tend à diminuer cette variabilité. Ainsi, après une à deux années de repiquages réguliers, on obtient une collection de souches stables présentant des caractéristiques de croissance et de production de métabolites très différentes.

A ce stade, il est alors possible de sélectionner, à l'aide de tests bien définis, la ou les souches produisant les composés d'intérêt en quantité importante. Le passage de ces cals en milieu liquide permet ensuite de s'orienter vers des volumes de production plus importants, tout d'abord en fioles de 250 ml, et plus tard en bio-réacteur (20 Litres et plus). Les suspensions cellulaires ainsi obtenues, formées d'agrégats et de cellules isolées, peuvent encore présenter une hétérogénéité (variabilité somaclonale). Une sélection supplémentaire est alors effectuée pour obtenir des lignées cellulaires hautement productrices. En complément de ce clonage, la production du métabolite d'intérêt peut également

être optimisée par la modification des conditions de culture conduisant à élaboration de milieux, dits milieux de production. Ce milieu est identique au milieu d'entretien des cellules à l'exception de la concentration en saccharose qui est multipliée par deux. Lors de leur culture dans un milieu de production, les lignées cellulaires hautement productrices de Vigne cépage Cabernet Sauvignon sont élicitées dix jours après l'inoculation, par la lumière UV 254 nm à l'aide d'une lampe Wilber-Lourmat T-30C (600 $\mu W/m^2$), mise à une distance de 1m en éclairage direct au-dessus des cellules pendant 10 minutes, ce qui induit une accumulation considérable des polyphénols, en particulier des stilbènes, dans les cellules. Ce moyen d'élicitation ne forme évidemment aucune impureté dans la culture cellulaire. En fin de culture, c'est-à-dire trois jours après l'élicitation, les cellules végétales sont filtrées pour éliminer le milieu de culture restant et sont rincées à l'eau froide (4°C). On obtient ainsi une biomasse fraîche d'environ 350 grammes par litre de culture Les cellules extraites sont ensuite séchées et broyées. Un séchage plus ou moins important est réalisé afin d'obtenir le broyat de cellules de Vigne dédifférenciées et élicitées in vitro, sous forme d'une suspension visqueuse ou d'un gel ou d'une poudre sensiblement sèche. En contrôlant la vitesse de séchage et la teneur en eau des cellules, avantageusement entre 3 et 10%, il est possible de ne pas détruire la membrane des cellules avant l'étape de broyage, ceci permet que les phytoalexines contenues dans les cellules, en particulier dans les vacuoles des cellules et/ou dans la membrane, soient libérées au moment de l'étape de broyage.. Dans le cas de cellules sensiblement sèche, on obtient après lyophilisation à l'aide d'un appareil Virtis (Uni-Trap 10-100) environ 20 grammes de biomasse sèche par litre de culture. La poudre obtenue après broyage à l'aide d'un broyeur type Mortier, est légère, ultrafine (taille des particules inférieure à 10 μm) et de couleur beige - clair.

Les cellules élicitées et séchées sont broyées pour former des particules présentant une granulométrie moyenne (en poids) comprise entre 1 et 10μm. Il est possible le cas échéant de soumettre le broyant à une séparation granulométrique (tamis, etc.) pour ne conserver que des particules d'une plage de granulométrie déterminée, par exemple de la plage 5 - 10μm, 1-5 μm, moins de 3μm, etc.

Les cellules dédifférenciées ont été préparées à partir de matières diverses et ont été élicitées au moyen d'agents divers. Ces données sont reprises dans le tableau suivant :

| Exemple 1 (vigne) | matière dont les cellules dédifférenciées proviennent | Type d'élicitation |
|---|---|---|
| 1A | rameau de moins d'un an d'age | rayon UV pendant 3 jours |
| 1B | cuticule de raisin mûr | gaz carbonique pendant 24 heures |
| 1C | cuticule de raisin vert | gaz carbonique pendant 2 jours |
| 1D | pépin de raisin | rayon UV et gaz carbonique pendant 5 jours |
| 1E | racine | rayon UV pendant 12 heures |
| 1F | feuille verte | rayon UV pendant 15 heures |
| 1G | bourgeon | rayon UV pendant 75 heures |
| 1H | résidu d'une étape de pressage | rayon UV pendant 2 jours |
| 1I | résidu d'une étape de pressage | rayon UV pendant 3 jours |

On notera de plus qu'il est intéressant d'effectuer l'opération de broyage des cellules dédifférentiées et élicitées en présence d'un ou de plusieurs agents ou excipients de la composition cosmétique de manière à assurer la libération des phytoalexines dans au moins, certains agents de la composition cosmétique.

Exemple 2 <u>Détermination par HPLC de la teneur en stilbènes dans des cellules de vigne élicitées (exemple 1A) et non élicitées.</u>

Matériels et méthodes:

- Pompe Bischoff Model 2.200
- Injecteur automatique Alcoot Model 788 autosampler
- Colonne Ultrasep C18 (30 cm x 0,18 cm) 6 mm de porosité
- Détecteur de fluorescence, Jasco 821-FI

La détection de la fluorescence a été effectuée avec une excitation à 300 nm et une émission à 390nm. L'éluant utilisé est composé de méthanol : eau, 40:60 (v/v) dont le pH 8,3 a été ajusté avec du KOH 1M.

Résultat:

La teneur en stilbènes est d'environ 1 % par rapport au poids de matière sèche des cellules de Vigne élicitées in vitro. La teneur en phytoalexines demeure inférieure à 0,05 % dans les cellules non élicitées. Cette teneur élevée en stilbène (20 fois plus) est un moyen de contrôle de l'étape d'élicitation, et donc du procédé de fabrication d'un broyat suivant l'invention. Vu la concentration importante en stilbène dans la broyat suivant l'invention, il est possible par des procédés d'extraction, de distillation, de crystallisation, etc. de produire des milieux encore plus concentrés en stilbènes ou phytoalexines, voire de produire des photoalexines sensiblement pures.
Les compositions selon l'invention présenteront donc un rapport teneur en phytoalexines/teneur en membrane cellulaire broyée important par rapport au rapport phytoalexines/teneur en membrane cellulaire broyée pour des cellules végétales non élicitées et/ou naturelles.

Exemple 3 <u>Activité pharmacologique d'un broyat de vigne : antioxydant</u>

L'activité anti-radicalaire du produit obtenu selon l'exemple 1A a été étudiée in vitro. Nous avons utilisé un modèle d'épidermes reconstitués SKINETHIC®, permettant de révéler cette activité par dosage du malondialdéhyde (MDA), après son induction par des rayons ultraviolets B.

Répartition des épidermes

L'essai a été conduit en triplicate, après 24 heures de contact du produit (broyat de l'exemple 1A) avec les épidermes. Des kératinocytes d'origine humaine sont ensemencés sur des filtres en polycarbonate de 0,63 $cm^2$ dans un milieu défini (MCDB 153 modifié) et supplémenté. Les cellules sont cultivées pendant 14 jours à l'interface air/ liquide, le milieu de culture étant changé tous les deux jours.

Les épidermes ainsi formés ont été utilisés pour la réalisation de l'étude à partir du 17ème jour de la culture.

Lots d'épidermes reconstitués SKTIVETHIC®:

- Lot 1 : 3 épidermes témoins ne recevant ni produit ni irradiation
- Lot 2 : 3 épidermes irradiés aux UVB (150 $mJ/cm^2$)
- Lot 3 : 3 épidermes traités avec la SOD+Catalase + UVB (150 $J/cm^2$)
- Lot 4 : 3 épidermes traités avec broyat (0,1%)
- Lot 5 : 3 épidermes traités avec broyat (0,5%)
- Lot 6 : 3 épidermes traités avec broyat (1%)
- Lot 7 : 3 épidermes traités avec broyat (0,1%) + UVB (150 $mJ/cm^2$)
- Lot 8 : 3 épidermes traités avec broyat (0,5%) + UVB (150 $mJ/cm^2$)
- Lot 9 : 3 épidermes traités avec broyat (1%) + UVB (150 $mJ/cm^2$)

Dosage du malondialdéhyde (MDA) : indice de lipopéroxydation

Extraction du malondialdéhyde (MDA)

24 heures après le traitement des épidermes reconstitués SKINETHIC®, les cellulaires ont été remises en suspension dans :

- 250 μl de tampon Tris 50 mM, pH 8 contenant NaCl 0,1M; EDTA 20 mM
- 25 μl de SDS à 7%
- 300 μl de HCl (0,1 N)
- 38 μl d'acide phosphotungstique à 1% dans l'eau
- 300 μl d'acide thiobarbiturique à 0,67% dans l'eau

Après 1 heure d'incubation dans l'obscurité à 50°C et un refroidissement dans l'eau glacée, 300 μl de n-butanol ont été ajoutés dans chaque tube. Ceux-ci ont été centrifugés à 10 000 g à 0°C pendant 10 minutes. La phase supérieure a été récupérée pour le dosage du MDA.

Dosage du malondialdéhyde (MDA)

Le MDA a été dosé par mesure de la fluorescence après séparation du complexe MDA-TBA par HPLC.

- Pompe Bischoff Model 2.200
- Injecteur automatique Alcoot Model 788 autosampler
- Colonne Ultrasep C 18 (30 cm x 0,18 cm) 6 mm de porosité
- Détecteur de fluorescence, Jasco 821-FI

La détection de la fluorescence a été effectuée avec une excitation à 515 nm et une émission à 553nm. L'éluant utilisé est composé de méthanol : eau, 40:60 (v/v) dont le pH 8,3 a été ajusté avec du KOH 1M.
La quantification a été faite par rapport à des standards traités comme les échantillons (0,125; 0,25; 0,5 et 1 μM) à l'aide d'un logiciel informatique ICS (Pic 3) (Instrumentation Consommable Service).

Dosage des protéines

Le dosage des protéines a été réalisé selon la méthode de BRADFORD. L'augmentation de l'absorbance à 595 nm est proportionnelle à la concentration des protéines déterminées à l'aide d'un spectrophotomètre UNICAM 8625.

Résultats

Dosage du malondialdéhyde (MDA) dans l'homogénat cellulaire
Les résultats sont regroupés dans le tableau ci-dessous :

| | MDA (μM/mg protéines) | Variation de MDA (en %) par rapport au témoin |
|---|---|---|
| Témoin | 546 ± 40,81 | - |
| broyat (0,1 %) | 445,3 ± 57,72* | - 18 (diminution) |
| broyat (0,5%) | 409,5 ± 48,58* | - 25(diminution) |
| broyat (1%) | 325,5 ± 28,85* | - 40(diminution) |
| * Significativement différent par rapport au témoin : p < 0,005 (Wilcoxon Rank Sum Test). | | |

Les résultats obtenus révèlent une protection du broyat significative vis-à-vis de la lipopéroxydation physiologique aux dilutions 0,1 ; 0,5 et 1% respectivement de 18%, 25% et 40%.

Lipopéroxydation provoquée

Les résultats sont regroupés dans le tableau ci-dessous :

| | MDA (μM/mg protéines) | Variation de MDA (en %) par rapport au témoin avec UVB |
|---|---|---|
| Témoin | 546 ± 40,81 | - |
| UVB (150 mJ/cm$^2$) | 792,4 ± 59,4 | (accroissement de 45% par rapport au témoin sans UVB) |
| SOD/Catalase + UVB (150 mJ/cm$^2$) | 482,5 ± 22,1 | -39(diminution) |
| broyat (0,1%) + UVB (150 mJ/cm$^2$) | 545 ± 43,4** | -31(diminution) |
| broyat (0,5%) + UVB (150 mJ/cm$^2$) | 485,5 ± 35,6** | -39(diminution) |
| broyat (1%) + UVB (150 mJ/cm$^2$) | 420,3 ± 46,3* | -47(diminution) |
| * Significativement différent par rapport au témoin p < 0,005 (Wilcoxon Rank Sum Test).<br>** Significativement différent par rapport au témoin p ≤ 0,05 (Wilcoxon Rank Sum Test). | | |

Les résultats obtenus révèlent une protection significative du broyat vis à vis de la lipopéroxydation provoquée par les rayons ultraviolets B (150 mJ/cm$^2$) aux concentrations de 0,1 ; 0,5 et 1% respectivement de 31%, 39% et 47%

comparativement aux enzymes protectrices SOD/catalase (39%).

Dans les conditions expérimentales retenues, le broyat est apparu présenter une activité anti-radicalaire significative au niveau des épidermes reconstitués SKIMETHIC® après 24 heures de contact. Cette activité a été révélée par le dosage du MDA.

En effet, les dosages du MDA montrent que le broyat étudié aux concentrations de 0,1 ; 0,5 et 1% :

- diminue significativement le taux du MDA physiologique respectivement de 18%, 25% et 40%.
- protège significativement les cellules contre la lipopéroxydation provoquée par les rayons ultraviolets B (UVB 150 $mJ/cm^2$) en diminuant le taux de MDA induit de 31%, 39% et 47% comparativement à la SOD-catalase qui diminue le taux de MDA de 39%.

En conclusion, le broyat présente un effet anti-radicalaire aussi bien dans des conditions physiologiques que dans les conditions d'induction par les rayonnements ultraviolets B. Il ressort de cet essai que le broyat présente un effet anti-radicalaire significatif.
Compte tenu du model retenu (épiderme reconstitué), il peut d'ores et déjà être envisagé d'utiliser broyat dans des préparations à usage topique à la dose minimale active de 0,1%.

Exemple 4 <u>Recherche</u> de <u>l'effet du produits sur la vitesse de respiration (consommation d'oxygène en nanoatome d'oxygène</u> par <u>million de cellules et par minute)</u>

Cette expérience a été réalisée selon 2 conditions différentes :

- Effet sur la vitesse de respiration basale cellulaire au niveau des cellules non perméabilisées et en présence du glucose pour évaluer la respiration cellulaire.
- Effet sur la vitesse de la respiration des cellules perméabilisées en présence du substrat respiratoire, pyruvate-malate, pour évaluer la respiration mitochondriale.

Cette étude a été réalisée sur des kératinocytes humains en culture dissociés à la trypsine. 5 à 10 millions de kératinocytes en culture ont été mis en suspension dans 1 ml de milieu Hanks-Hepes à 30°C contenant du glucose (20 mM). La respiration a été suivie en temps réel et exprimée en nanoatomes d'oxygène consommés par minute et par $10^6$ cellules. L'addition de différentes quantités du produit dans la cuve de l'oxygraphe a permis de mettre en évidence une éventuelles stimulation ou inhibition de la respiration.

La quantité d'oxygène dissous dans un milieu d'incubation a été déterminée à l'aide d'une électrode de Clark. L'oxygène qui diffuse à travers un film de téflon est réduit au niveau de la cathode de platine polarisée à -0.8 Volt. Dans ces conditions le courant passant entre cette cathode et l'anode d'argent est proportionnel à la concentration en oxygène dans la solution. Le pont ionique est assuré par une solution de KCl demie saturée. L'acquisition et le traitement des mesures sont faits sur un micro-ordinateur.

<u>- Effet sur la vitesse de la respiration basale cellulaire</u>

Cet essai a été effectué sur des cellules entières non perméabilisés en présence du glucose.

Les essais ont été réalisés à partir d'une solution mère du produit à 0,3%.

Les résultats sont regroupés dans le tableau ci-dessous :

Vitesse de la respiration basale cellulaire

| broyat (μl de produit) | 0.00 | 2 | 5 | 10 | 50 | 100 |
|---|---|---|---|---|---|---|
| Respiration basale (natomes/minute/ $10^6$ cel.) (n=3) | 1,15 ± 0,5 | 1,22±0,21 | 1,31±0,14 | 1,58± 0,64 | 2,30± 0,27 | 2,58± 0,21 |
| % de la respiration cellulaire basale | 100 | 106 | 114 | 137 | 200 | 224 |

<u>- Effet sur la vitesse de la respiration mitochondriale</u>

Cet essai a été réalisé sur les cellules perméabilisées en présence du substrat respiratoire, pyruvate-malate.

Les résultats sont regroupés dans le tableaux ci-dessous.

Vitesse de respiration mitochondriale en présence du pyruvate-malate.

| Broyat (µl de produit) | 0.00 | 2 | 5 | 10 | 50 | 100 |
|---|---|---|---|---|---|---|
| Respiration pyruvate (natomes/minute/ $10^6$ cel.) (n=3) | 1,58± 0,12 | 1,98± 0,05 | 2,05± 0,14 | 2,25± 0,42 | 2,58± 0,22 | 3,02± 0,38 |
| % de la respiration pyruvate | 100 | 125 | 130 | 142 | 163 | 191 |

Les résultats montrent que le broyat, à différentes doses, augmente la vitesse de la respiration (consommation d'oxygène) aussi bien au niveau des cellules entières non perméabilisées (en présence de glucose), traduisant une augmentation de la respiration basale cellulaire, qu'au niveau des cellules perméabilisées (en présence du pyruvate-malate), traduisant une augmentation de la respiration mitochondriale.

Exemple 5 <u>Recherche de l'effet du produit sur la vitesse de</u> synthèse <u>d'ATP en moles par million de cellules et par minute.</u>

Cette étape a été réalisée selon 2 conditions différentes :

- Effet sur la vitesse de synthèse d'ATP au niveau des cellules non perméabilisées et en présence du glucose pour évaluer la vitesse de synthèse cellulaire.
- Effet sur la vitesse de synthèse d'ATP au niveau des cellules perméabilisées en présence du substrat respiratoire, pyruvate-malate, pour évaluer la vitesse dé synthèse mitochondriale.

Cette étude a été réalisée sur des kératinocytes humains en culture dissociés à la trypsine. 5 à 10 millions de kératinocytes en culture sont mis en suspension dans 1 ml de milieu Hanks-Hepes à 30°C contenant du glucose (20 mM). L'addition de différentes quantités du produit dans la cuve a permis de mettre en évidence une éventuelle activation ou inhibition de la vitesse de synthèse d'ATP.

La quantité d'ATP présente dans le milieu a été déterminée grâce à la réaction enzymatique suivante :

$$ATP + LUCIFERINE \xrightarrow[Mg^{2+}]{Luciférase} OXYLUCIFERINE + AMP + Ppi + CO_2 + h\nu$$

Elle s'effectue dans un appareil de type Luminoscan en utilisant l'ATP monitoring reagent (ATP Bioluminescence Assay Kit HS II) de Boehringer Mannheim.

L'intensité de la lumière émise lors de cette réaction a été mesurée par un luminomètre (Luminoscan) qui la transcrit en RLU (unité relative de luminosité). Les RLU mesurées ont été converties en mole d'ATP en se référant à une gamme étalon d'ATP .

<u>- Effet sur la vitesse de synthèse d'ATP : taux de synthèse cellulaire basale</u>

Cet essai a été réalisé sur des cellules entières non perméabilisées en présence de glucose. Les résultats sont regroupés dans le tableau ci-dessous.

Vitesse de synthèse d'ATP

| BROYAT (µl de produit) | 0.00 | 2 | 5 | 10 | 50 | 100 |
|---|---|---|---|---|---|---|
| Vitesse de synthèse (nmolesATP/mn/$10^6$ cel.) (n=3) | 2,58± 0,15 | 2,65± 0,12 | 3,08± 0,22 | 3,12± 0,30 | 5,80± 0,95 | 5,95± 0,72 |
| % de synthèse cellulaire | 100 | 103 | 119 | 121 | 225 | 231 |

<u>- Effet sur la vitesse de synthèse d'ATP mitochondriale</u>

Vitesse de synthèse d'ATP mitochondriale en présence du pyruvate-malate.

| BROYAT (µl de produit) | 0.00 | 2 | 5 | 10 | 50 | 100 |
|---|---|---|---|---|---|---|
| Vitesse de synthèse (nmolesATP/mn/$10^6$ cel.) (n=3) | 4,15± 0,22 | 4,95± 0,16 | 4,95± 0,18 | 5,02± 0,16 | 5,75± 0,28 | 7,58± 0,85 |
| % de synthèse mitochondriale | 100 | 119 | 119 | 121 | 139 | 183 |

Les résultats montrent que le broyat, à différentes doses, augmente la vitesse de synthèse d'ATP aussi bien au niveau des cellules entières non perméabilisées (en présence du glucose), traduisant une augmentation de la synthèse d'ATP cellulaire, qu'au niveau des cellules perméabilisées (en présence du pyruvate-malate) traduisant une augmentation de la synthèse mitochondriale.

Exemple 6 <u>Recherche de l'effet du produit sur le métabolisme énergétique des cellules en cultures, Dosage des nucléotides adényliques cellulaires (ATP, ADP et AMP) en nmoles/mg de protéines, et calcul de la charge énergétique (CE) des cellules traitées 5 jours par le produit.</u>

Les kératinocytes humains ont été mis en culture durant 5 jours en absence et en présence du produit ($10^7$ cellules par mesure).

Une fois trypsinisées, les cellules ont été récoltées et les concentrations des nucléotides adényliques ont été déterminées par HPLC.

Cet essai a été réalisé sur des cellules entières non perméabilisées.

Les résultats sont regroupés dans le tableau ci-dessous.

| Doses | [ATP] | [ADP] | [AMP] | [ATP/ADP] | Somme | C.E. |
|---|---|---|---|---|---|---|
| contrôle | 4222 | 1014 | 748 | 4.16 | 5984 | 0.79 |
| 0,02% | 4532 | 1435 | 837 | 3.16 | 6804 | 0.77 |
| 0,05% | 5292 | 1327 | 779 | 3.99 | 7398 | 0.80 |
| 0,1% | 6184 | 1231 | 796 | 5.02 | 8211 | 0.83 |
| 0,5% | 6848 | 2195 | 978 | 3.12 | 10021 | 0.79 |
| 1% | 7791 | 2532 | 1131 | 3.08 | 11454 | 0.79 |

Les concentrations en ATP, ADP et AMP sont exprimées en nmoles/mg protéines (n = 3).

$$\text{Somme} = [\text{ATP}] + [\text{ADP}] + [\text{AMP}]$$

$$\text{Charge énergétique (C.E.)} = [\text{ATP}] + 1/2[\text{ADP}]/([\text{ATP}] + [\text{ADP}] + [\text{AMP}])$$

Les résultats montrent que le broyat, à différentes doses, augmente d'une façon dose-dépendante :

- la concentration de l'ATP synthétisé,
- la concentration totale des nucléotides adényliques cellulaires.

Par ailleurs, la charge énergétique (CE) a demeuré constante, traduisant un équilibre énergétique stable entre les nucléotides adényliques :

ATP → ADP + Pi
ATP → AMP + PPi
(Pi : phosphate inorganique)

Ces résultats sont en parfaite corrélation avec ceux obtenus au cours des 2 premières étapes, et confirment

bien que le produit entraîne une stimulation du métabolisme énergétique cellulaire.

Exemple 7 Etude de la tolérance d'un broyat de vigne

Une étude de tolérance cutanée et oculaire in vitro à été réalisée à titre préliminaire en vue de l'élaboration de préparations cosmétiques.
Ces essais ont révélé une parfaite tolérance locale (cutanée et oculaire) du broyat à la concentration de 0,3%.
Les broyats de cellules peuvent être directement utilisés pour former des compositions cométiques à usage topique.
On donnera ci-après quelques exemples, non limitatifs, de telles compositions à usage topique

Exemple 8 Dispersion de cellules de vigne élicitées et entières dans une base cosmétique

Les cellules de vigne sont obtenues comme décrit dans les exemples 1A à 1I. Les cellules de ces exemples ont été utilisées séparément ou en mélange pour la préparation d'une composition cosmétique. Les cellules sont dispersées après lyophilisation sans avoir été broyées dans la base suivante :

- Eau déionisée 85,61 %
- Huile minérale 9,00 %
- Alcool cétylique 3, 00 %
- ceteareth - 20 0, 75 %
- cellules de vigne 0, 20 %
- fragrance 0, 15 %
- carbomer 0, 10 %
- méthylchloroisothiazoline
  et méthylisothiazoline [kathon CG] 0, 065 %
- hydroxyde de sodium (45 %) 0, 06 %
- hydroxyanisole butylée 0, 06 %

TOTAL 100, 00 %

La composition obtenue montre une dispersion homogène des cellules dans la crème et une granulométrie très fine. L'étude de propreté a montré l'absence de germes et champignons ainsi qu'une remarquable stabilité de la composition. Le résultat obtenu ayant été testé dans une étude transcutanée a permis de voir le passage des principes actifs notamment les polyphénols à travers le tissu cutané.

Exemple 9 Dispersion de cellules de vigne élicitées et broyées dans une base cosmétique

Les cellules de vigne sont obtenues comme décrit dans les exemples 1A à 1I. Les cellules de ces exemples ont été utilisées séparément ou en mélange pour la préparation d'une composition cosmétique. Les cellules sont dispersées après lyophilisation et broyage dans la base suivante :

- eau déionisée 85, 61 %
- huile minérale 9,00 %
- alcool cétylique 3,00 %
- ceteareth - 20 0, 75 %
- cellules de vigne 0,20 %
- fragrance 0, 15 %
- carbomer 0, 10 %
- méthylchloroisothiazoline
  et méthylisothiazoline [kathon CG] 0, 065 %
- hydroxyde de sodium (45 %) 0, 06 %
- hydroxyanisole butylée 0, 06 %

TOTAL 100,00 %

La composition obtenue montre une dispersion homogène du broyat de cellules dans la crème et une granulométrie très fine. L'étude de propreté a montré l'absence de germes et champignons ainsi qu'une remarquable stabilité de la composition. Le résultat obtenu ayant été testé dans une étude transcutanée a permis de voir le passage des principes actifs notamment les polyphénols à travers le tissu cutané.

Exemple 10 Dispersion de cellules de vigne élicitées et entières dans une base cosmétique

Les cellules de vigne sont obtenues comme décrit dans les exemples 1A à 1I. Les cellules de ces exemples ont été utilisées séparément ou en mélange pour la préparation d'une composition cosmétique. Les cellules sont dispersées après lyophilisation sans avoir été broyées dans la base suivante :

- eau 46,89 %
- laureth sulfate de sodium (25%) 36, 40 %
- PEG-7 glyceryl cocoate 2, 00 %
- laureth-2 1, 50 %
- laureth-11 carboxylate de sodium 4, 00 %
- cocamidopropyl bétaine & acide benzoique 3, 48 %
- chlorure de sodium 1, 60 %
- propylène glycol 1, 00 %
- parfum 0, 13 %
- PEG-40 huile hydrogénée de castor & propylène glycol & eau 0, 50 %
- oleth-10 0, 50 %
- phosphate de sodium 0, 30 %
- phosphate dissodique 0, 08 %
- acide citrique (50 %) 0, 52 %
- benzoate de sodium 0, 50 %
- cellules de vigne 0, 20 %
- acide salicylique 0, 20 %
- phénoxyéthanol 0, 20 %

TOTAL 100, 00 %

La composition obtenue montre une dispersion homogène des cellules dans la crème et une granulométrie très fine. L'étude de propreté a montré l'absence de germes et champignons ainsi qu'une remarquable stabilité de la composition. Le résultat obtenu ayant été testé dans une étude transcutanée a permis de voir le passage des principes actifs notamment les polyphénols à travers le tissu cutané.

Exemple 11 Dispersion de cellules de vigne élicitées et broyées dans une base cosmétique

Les cellules de vigne sont obtenues comme décrit dans les exemples 1A à 1I. Les cellules de ces exemples ont été utilisées séparément ou en mélange pour la préparation d'une composition cosmétique. Les cellules sont dispersées après lyophilisation et broyage dans la base suivante :

- eau 46,89 %
- laureth sulfate de sodium (25%) 36, 40 %
- PEG-7 glyceryl cocoate 2, 00 %
- laureth-2 1, 50 %
- sodium laureth-11 carboxylate 4, 00 %
- cocamidopropyl bétaine & acide benzoique 3, 48 %
- chlorure de sodium 1, 60 %
- propylène glycol 1, 00 %
- parfum 0, 13 %
- PEG-40 huile hydrogénée de castor& propylène glycol & eau 0, 50 %
- oleth-10 0, 50 %
- phosphate de sodium 0,30 %
- phosphate disodique 0, 08 %
- acide citrique (50 %) 0, 52 %
- benzoate de sodium 0, 50 %
- broyat de cellules de vigne 0, 20 %
- acide salicylique 0, 20 %
- phénoxyéthanol 0, 20 %

TOTAL 100,00 %

La composition obtenue montre une dispersion homogène du broyat de cellules dans la crème et une granulométrie très fine. L'étude de propreté a montré l'absence de germes et champignons ainsi qu'une remarquable stabilité de la composition. Le résultat obtenu ayant été testé dans une étude transcutanée a permis de voir le passage des principes actifs notamment les polyphénols à travers le tissu cutané.

Exemple 12 Crèmes

- phase aqueuse A : eau déminéralisée associée à un produit hydratant
- phase huileuse B : émulsionnant + émolliant + huile
- phase C : conservateur, parfum
- phase D : produit actif : broyat de cellules de Vigne dédifférenciées et élicitées, sous forme d'une suspension visqueuse ou d'un gel ou d'une poudre sensiblement sèche.

Exemple 13 Lotions

contenant seulement une phase aqueuse A : eau déminéralisée, propylène glycol, conservateur, parfum et produit actif : broyat de cellules de Vigne dédifférenciées et élicitées, sous forme d'une suspension visqueuse ou d'un gel ou d'une poudre sensiblement sèche.

Exemple 14 Shampooings

contenant seulement une phase aqueuse A à base d'eau déminéralisée, détergents, moussants, épaississants, parfum et produit actif : broyat de cellules de Vigne dédifférenciées et élicitées, sous forme d'une suspension visqueuse ou d'un gel ou d'une poudre sensiblement sèche.

Exemple 15 Gels.

- On considère les hydrogels et les oléogels, obtenus par adjonction à la phase aqueuse A ou à la phase huileuse B d'agents de type émulsifiant et épaississant
- phase C : parfum, conservateur
- phase D : broyat de cellules de Vigne dédifférenciées et élicitées, sous forme d'une suspension visqueuse ou d'un gel ou d'une poudre sensiblement sèche.

Exemple 16 Solutions

Solutions contenant seulement une phase aqueuse A essentiellement à base d'eau déminéralisée, parfum, conservateur et produit actif : broyat de cellules de Vigne dédifférenciées et élicitées, sous forme d'une suspension visqueuse ou d'un gel ou d'une poudre sensiblement sèche.

Exemple 17 Laits

- phase aqueuse A : essentiellement à base d'eau désionisée
- phase huileuse B : huile + émulsionnant + émolliant
- phase C : conservateur + produit hydratant
- phase D : produit actif : broyat de cellules de Vigne dédifférenciées et élicitées, sous forme d'une suspension visqueuse ou d'un gel ou d'une poudre sensiblement sèche.

Dans les exemples donnés ci-dessus, concernant les crèmes, gels ou laits, les différentes phases A, B, C et D, dans des proportions qui peuvent varier entre elles en fonction des applications souhaitées, sont mélangées de manière habituelle, comme le réalise habituellement l'Homme de l'art dans ce domaine.

Concernant les lotions, solutions et shampooings, la composition à usage topique contient les différents constituants, dont on peut varier les teneurs en fonction des applications, mélangés dans la seule phase aqueuse, comme

le réalise habituellement l'Homme de l'art dans ce domaine.

La proportion du broyat de cellules de Vigne dédifférenciées et élicitées, sous forme d'une suspension visqueuse ou d'un gel ou d'une poudre sensiblement sèche est fonction de la nature de la composition à usage topique et de l'application souhaitée. Elle est avantageusement comprise entre 0,01 et 5 %, mais peut atteindre jusqu'à 25 %.

Evidemment, l'invention n'est pas limitée aux exemples de réalisation donnés ci-dessus et il est possible de réaliser la composition à usage topique sous d'autres formes, telles que huile, onguent, laques, fards (fond de teint, poudre, rouge à lèvres, crayon, mascara, ombre à paupières) qui entrent aussi dans le cadre de l'invention.

Egalement, l'invention n'est pas limitée aux cellules de Vigne et peut s'appliquer à d'autres types de cellules végétales, du moment qu'elles puissent être obtenues sous forme dédifférenciées et qu'elles puissent subir une élicitation menant à une accumulation de métabolites secondaires en quantité suffisante pour permettre l'activité biologique en usage topique.

Dans tous les cas, on obtient une composition à usage topique qui contient un broyat de cellules végétales dédifférenciées et élicitées, sous forme d'une suspension visqueuse ou d'un gel ou d'une poudre sensiblement sèche.

<u>Exemple 18</u>

On a répété l'exemple 1, en utilisant des cellules végétales dédifférenciées provenant d'espèces végétales différentes ou de mélanges d'espèces végétales différentes. Dans ces exemples, on a utilisé des écorces, des pépins ou des graines, des racines , des feuilles, des tiges, des bourgeons, des fruits, peau ou cuticule pour obtenir des cellules végétales dédifférenciées.

Le tableau suivant reprend les espèces végétales utilisées :

| Exemple 18 | Espèces végétales |
|---|---|
| A | Rosmarinus |
| B | Coffea |
| C | Cacao |
| D | Mungo |
| E | Colchicum |
| F | Jasminum + Iris |
| G | Capsicum |
| H | Pilocarpus |
| I | Sequoia |
| J | Solanum |
| K | Chlorophytum |
| L | Ginkgo |
| M | digitalis |
| N | Salvia |
| O | Taxus |
| P | Papaver |
| Q | Salvia + rosmarinus |
| R | Roses |
| S | thé |
| T | Bétula |
| U | Vigne + citrus + ginko |

Exemple 19 Activité antioxydante de broyats de différentes éspèces végétales :

L'activité anti-radicalaire de broyats de broyats issus de différentes éspèces végétales a été étudiée in vitro. Nous avons utilisé un modèle d'épidermes reconstitués SKINETHIC®, permettant de révéler cette activité par dosage du malondialdéhyde (MDA), après son induction par des rayons ultraviolets B. Les épidermes ont été traité pour chaque espèce végétale par une concentration unique de broyat à 1%.

Lipopéroxydation provoquée

Les conditions expérimentales de l'exemple 3 ont été répétées.
Les résultats sont regroupés dans le tableau ci-dessous :

| Espèce végétale, broyat à 1% | Variation du MDA (en %) par rapport au témoin négatif |
|---|---|
| Témoin négatif | - |
| Témoin positif UVB (150 $mJ/cm^2$) | accroissement de 45% |
| Cacao broyat à 1%+ UVB (150 $mJ/cm^2$) | -29 % |
| Mungo broyat à 1%+ UVB (150 $mJ/cm^2$) | -22 % |
| Colchicum broyat à 1%+ UVB (150 $mJ/cm^2$) | -26 % |
| Jasminum broyat à 1%+ UVB (150 $mJ/cm^2$) | -17 % |
| Capsicum broyat à 1%+ UVB (150 $mJ/cm^2$) | -43 % |
| Pilocarpus broyat à 1%+ UVB (150 $mJ/cm^2$) | -41 % |
| Sequoia broyat à 1%+ UVB (150 $mJ/cm^2$) | -59 % |
| Solanum broyat à 1%+ UVB (150 $mJ/cm^2$) | -19 % |
| Chlorophytum broyat à 1%+ UVB (150 $mJ/cm^2$) | -53 % |
| Ginkgo broyat à 1%+ UVB (150 $mJ/cm^2$) | -52 % |
| Roses broyat à 1% + UVB (150$mJ/cm^2$) | -25 % |
| Betula broyat à 1% + UVB (150 $mJ/cm^2$) | -30 % |
| digitalis broyat à 1%+ UVB (150 $mJ/cm^2$) | -33 % |
| Salvia broyat à 1%+ UVB (150 $mJ/cm^2$) | -47 % |
| Taxus broyat à 1%+ UVB (150 $mJ/cm^2$) | -56 % |
| Papaver broyat à 1%+ UVB (150 $mJ/cm^2$) | -53 % |
| Cannabis broyat à 1%+ UVB (150 $mJ/cm^2$) | - 47 % |
| Rosmarinus broyat à 1%+ UVB (150 $mJ/cm^2$) | -27 % |
| Coffea broyat à 1%+ UVB (150 $mJ/cm^2$) | -34 % |
| Arganier broyat à 1%+ UVB (150 $mJ/cm^2$) | -51 % |
| Catharantus broyat à 1%+ UVB (150 $mJ/cm^2$) | -59 % |
| Iris broyat à 1%+ UVB (150 $mJ/cm^2$) | -19 % |
| Datura broyat à 1%+ UVB (150 $mJ/cm^2$) | -53 % |
| Gloriosa broyat à 1%+ UVB (150 $mJ/cm^2$) | -12 % |
| Medicago broyat à 1%+ UVB (150 $mJ/cm^2$) | -33 % |
| Asparagus broyat à 1%+ UVB (150 $mJ/cm^2$) | -47 % |
| Borago broyat à 1%+ UVB (150 $mJ/cm^2$) | -22 % |
| Reseda broyat à 1%+ UVB (150 $mJ/cm^2$) | -13 % |

(suite)

| Espèce végétale, broyat à 1% | Variation du MDA (en %) par rapport au témoin négatif |
|---|---|
| Amsonia broyat à 1%+ UVB (150 mJ/cm$^2$) | -26 % |
| Erythrina broyat à 1%+ UVB (150 mJ/cm$^2$) | -21 % |
| Coleus broyat à 1%+ UVB (150 mJ/cm$^2$) | -53 % |
| Oenothera broyat à 1%+ UVB (150 mJ/cm$^2$) | -17 % |
| Atropa broyat à 1%+ UVB (150 mJ/cm$^2$) | -23 % |
| Theobroma broyat à 1%+ UVB (150 mJ/cm$^2$) | -17 % |
| Glycine broyat à 1%+ UVB (150 mJ/cm$^2$) | -43 % |
| psoralea coryilfolia broyat à 1%+UVB150mJ/cm$^2$ | -40 % |
| vitex negundo broyat à 1%+ UVB (150 mJ/cm$^2$) | - 42 % |
| commiphora wighii broyat à 1%+ UVB 150 J/cm$^2$ | - 51 % |
| vanilla planifolia broyat à 1%+ UVB (150 J/cm$^2$) | -14 % |
| marrubium vulgare broyat à 1%+UVB150mJ/cm$^2$ | - 28 % |
| pilocarpus jaborandi broyat 1%+UVB150mJ/cm$^2$ | -41 % |

Les résultats obtenus révèlent une protection significative des broyats vis à vis de la lipopéroxydation provoquée par les rayons UVB (150 mJ/cm$^2$) aux concentrations de 1%.

Dans les conditions expérimentales retenues, les broyats sont apparus présenter une activité anti-radicalaire significative au niveau des épidermes reconstitués SKINETHIC® après 24 heures de contact. Cette activité a été révélée par le dosage du MDA.

En effet, les dosages du MDA montrent que les broyats étudiés aux concentrations de 1% protègent significativement les cellules contre la lipopéroxydation provoquée par les rayons ultraviolets B (UVB 150 mJ/cm$^2$) en diminuant les taux de MDA induits.

En conclusion, les broyats des différentes espèces végétales étudiées présentent un effet anti-radicalaire dans les conditions d'induction par les rayonnements ultraviolets B. Il ressort de cet essai que les broyats présentent un effet anti-radicalaire significatif. Compte tenu du modèle retenu (épiderme reconstitué), il peut d'ores et déjà être envisagé d'utiliser ces broyats dans des préparations à usage topique à la dose minimale active de 0,1%.

Exemple 20

Lipopéroxydation provoquée

Les conditions expérimentales de l'exemple 3 ont été répétées en utilisant la combinaison de plantes aromatiques avec d'autres éspèces.

Les résultats sont regroupés dans le tableau ci-dessous :

| Espèce végétale, broyat à 1% | Variation du MDA (en %) par rapport au témoin négatif |
|---|---|
| Témoin négatif | - |
| Témoin positif UVB (150 mJ/cm$^2$) | accroissement de 45% |
| Jasminum sanbac+ Ginko bilboa broyat 1%+UVB150mJ/cm$^2$ | -12 % |
| eucalyptus punctata+ psoralea coryfolia broyat 1%+UVB150mJ/cm$^2$ | - 21 % |
| lavandula angustifolia + Vitex negundo broyat 1%+UVB150mJ/cm$^2$ | - 17 % |
| citrus limon + sequoia broyat 1 %+UVB150mJ/cm$^2$ | - 24 % |

Exemple 21

Les exemples 1A à 1I ont été répétés si ce n'est que les cellules dédifférenciées, élicitées et lavées ont été séchées dans une atmosphère (par exemple d'azote) présentant une température d'environ 30°C, de manière à réduire la teneur en eau des cellules à respectivement 5% en poids, 10% en poids et 15% en poids. La structure membranaire de la cellule a ainsi été préservée.
On a ensuite soumis les cellules dédifférenciées élicitées, lavées et séchées à un broyage.

Exemple 22

On a répété l'exemple 20, si ce n'est qu'on a mélangé les cellules dédifférenciées élicitées, lavées et séchées (avec la structure membranaire conservée) à un ou plusieurs excipients et/ou composés actif d'une composition cosmétique avant l'étape de broyage.
La liste suivante reprend certains excipients mélangés aux cellules avant leur broyage.

- sodium laureth sulfate (solution aqueuse par exemple à 25%)
- PEG-7 glyceryl cocoate
- antioxydant (solution aqueuse contenant de la vitamine E)
- laureth-2
- sodium laureth-11 carboxylate
- cocamidopropyl betaine
- betaine (glycinebetaine)
- huile essentielle
- propylene glycol
- ethanol
- PEG-40 hydrogenated castor oil
- huile végétale (huile de coco, huile d'olive, etc.)
- huile essentielle
- mélanges d'un ou de plusieurs composés cités ci-avant entre eux et/ou avec de l'eau.

La quantité d'excipients et/d'eau ajoutée aux cellules peut varier par exemple entre 10% du poids des cellules séchées à broyer et 100% dudit poids, voire plus.
L'utilisation d'un ou d'agents tensio actifs avec un ou plusieurs agents anti oxydants semble intéressante pour faciliter le broyage des cellules en particulier de la membrane, pour faciliter la libération de phytoalexines attachées ou liées à une membrane, et pour réduire ou éviter tout problème de dégradation de composés due à l'humidité. Il est ensuite possible d'extraire de ce broyat des phytoalexines par une étape d'extraction (par exemple étapes successives d'extraction au moyen d'éthanol et de filtration).
Cet exemple est donc un exemple de procédé d'obtention de phytoalexine(s), dans lequel :

- on met dans un milieu de culture des cellules végétales dédifférenciées,
- après et/ou pendant la culture, on élicite les cellules dédifférentiées dans le milieu de culture,
- on sépare les cellules dédifférentiées et élicitées du milieu de culture,
- on soumet éventuellement les cellules dédifférenciées élicitées à un ou plusieurs lavages,
- on sèche avantageusement, de préférence on lyophilise, les cellules dédifférenciées et élicitées,
- on broie les cellules dédifférenciées, élicitées de manière à former un broyat, et
- on soumet le broyat à une extraction pour extraire une ou plusieurs phytoalexines du broyat, éventuellement après une étape de remise des cellules broyées dans un milieu, en particulier un milieu aqueux et/ ou alcoolique.

Exemple 23

On a répété les exemples 1A à 1I, si ce n'est que les cellules dédifférenciées et élicitées ont été lavées et séchées (au moyen d'un courant d'azote à 30°C) pour éliminer l'eau de lavage présente à l'extérieure des membranes des cellules et pour réduire la teneur en eau présente dans les cellules, respectivement de 0%, 10%,25%,50% et 75%. Les cellules ont ensuite été broyées. Le broyat ainsi obtenu contient sensiblement tous les composants présents dans les cellules, avec une teneur en eau soit réduite, soit correspondant à l'eau présente dans les cellules normales (teneur normale en eau des cellules).

## Revendications

1. Composition pour application topique, en particulier composition cosmétique, contenant des cellules végétales dédifférenciées, **caractérisée en ce que** la composition contient au moins un broyat de cellules végétales dédifférenciées et élicitées en culture in vitro d'une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces,
pour synthétiser au moins une phytoalexine, cette élicitation pour synthétiser au moins une phytoalexine étant avantageusement opérée après une étape de cultures in vitro de cellules végétales sans élicitation, dans laquelle ledit broyat contenant au moins une phytoalexine comprend au moins 95%, avantageusement au moins 97%, de préférence au moins 99% en poids de l'ensemble des matières sèches issues des cellules végétales broyées dédifférenciées et élicitées in vitro, ledit broyat étant dispersé dans ladite composition ou étant sous une forme apte à être dispersée dans ladite composition.

2. Composition suivant la revendication 1, **caractérisée en ce que** le broyat de cellules végétales dédifférenciées et élicitées in vitro comprend des particules provenant des vacuoles, des particules provenant du cytoplasme et des particules provenant de la membrane pecto cellulosique, ledit broyat contenant au moins 0,1 % en poids de phytoalexine(s).

3. Composition suivant la revendication 1 ou 2, **caractérisée en ce que** le broyat est un broyat de cellules dédifférenciées et élicitées in vitro, lesdites cellules étant au moins partiellement séchées.

4. Composition suivant la revendication 3, **caractérisée en ce que** le broyat est un broyat de cellules dédifférenciées et élicitées in vitro, lesdites cellules étant sensiblement complètement séchées, de préférence lyophilisées, et ensuite broyées.

5. Composition selon l'une quelconque des revendications 1 à 4, **caractérisée en ce qu'**elle contient de 0,005 à 25% en poids, avantageusement de 0,005 à 5 % en poids de broyat de cellules végétales dédifférenciées et élicitées in vitro, ce poids étant calculé sous forme sèche.

6. Composition suivant l'une des revendications 1 à 5, **caractérisée en ce qu'**elle contient un broyat sec ou sensiblement sec de cellules végétales dédifférenciées et élicitées in vitro, ledit broyat sec ou sensiblement sec contenant une teneur en eau inférieure à 25% en poids, avantageusement inférieure à 15% en poids, de préférence inférieure à 10% en poids.

7. Composition suivant l'une des revendications 1 à 6, **caractérisée en ce que** le broyat a une granulométrie moyenne de particules solides inférieure à 100 $\mu$m, avantageusement inférieure à 10 $\mu$m, de préférence inférieure à 1 $\mu$m.

8. Composition suivant la revendication 7, **caractérisée en ce que** les particules du broyat ont une granulométrie telle que 90% en poids des particules ont une granulométrie comprise dans la plage granulométrie moyenne - 25% jusque granulométrie moyenne + 25%.

9. Composition suivant l'une quelconque des revendications précédentes, **caractérisée en ce que** ledit broyat de cellules végétales dédifférenciées et élicitées in vitro contient au moins une phytoalexine synthétisée par l'élicitation in vitro des cellules végétales dédifférenciées.

10. Composition suivant l'une quelconque des revendications précédentes, **caractérisée en ce que** ledit broyat de cellules végétales dédifférenciées et élicitées est un broyat de cellules végétales dédifférenciées et élicitées in vitro par un agent dans le milieu de culture, ledit broyat étant sensiblement exempt dudit agent après élicitation.

11. Composition suivant la revendication précédente, **caractérisée en ce que** les cellules dédifférenciées sont élicitées in vitro par un agent volatile.

12. Composition suivant l'une quelconque des revendications précédentes, **caractérisée en ce que** ledit broyat de cellules végétales dédifférenciées et élicitées in vitro contient au moins un composé terpénique ou tannique ou

polyphénolique, ledit composé étant synthétisé par l'élicitation in vitro des cellules végétales dédifférenciées dans leur milieu de culture.

**13.** Composition suivant l'une quelconque des revendications précédentes, **caractérisée en ce que** le broyat de cellules végétales dédifférenciées et élicitées in vitro se présente sous la forme d'une suspension visqueuse ou d'un gel ou d'une poudre sensiblement sèche, ladite suspension, gel ou poudre étant avantageusement sous une forme apte à être dispersée dans la composition.

**14.** Composition suivant l'une quelconque des revendications précédentes, **caractérisée en ce que** le broyat de cellules est un broyat de cellules de vigne dédifférenciées et élicitées in vitro.

**15.** Composition suivant l'une quelconque des revendications précédentes, **caractérisée en ce qu'**elle contient un broyat de cellules dédifférenciées, cultivées et élicitées dans leur milieu de culture in vitro, ledit broyat étant avantageusement sensiblement exempt de milieu de culture.

**16.** Composition suivant l'une quelconque des revendications précédentes, **caractérisée en ce qu'**elle contient un broyat de cellules dédifférenciées et élicitées in vitro, ledit broyat contenant au moins 0,1 % en poids de stilbènes par rapport au poids sec des cellules dédifférenciées élicitées broyées, en particulier au moins 0,2 % en poids de stilbènes par rapport au poids sec des cellules dédifférenciées élicitées broyées, de préférence au moins 0,5 % en poids de stilbènes par rapport au poids sec des cellules dédifférenciées élicitées broyées.

**17.** Procédé de préparation d'une composition à usage topique selon l'une quelconque des revendications précédentes, **caractérisé en ce que** l'on met des cellules végétales dédifférenciées d'au moins une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces, dans un milieu de culture de manière à permettre une croissance des cellules, **en ce que** l'on élicite lesdites cellules végétales différenciées dans leur milieu de culture pendant une période de temps suffisante pour la synthèse d'une quantité suffisante de métabolites, et **en ce qu'**on mélange les cellules végétales élicitées du milieu de culture avec un ou plusieurs excipients pour préparer une composition cosmétique, les cellules étant broyées avant et/ou après leur mélange avec un ou plusieurs excipients et/ou avant et/ou après une étape de séchage.

**18.** Procédé suivant la revendication 17, **caractérisé en ce qu'**on soumet lesdites cellules élicitées in vitro à un séchage, suivi d'un broyage.

**19.** Procédé suivant l'une quelconque des revendications 17 et 18, **caractérisé en ce qu'**on élicite les cellules dans leur milieu de culture in vitro au moyen d'un agent, qui après extraction des cellules élicitées du milieu de culture en conservant la structure membranaire des cellules ne se retrouve pas dans les cellules élicitées.

**20.** Procédé dans lequel des cellules végétales dédifférenciées sont mises en culture in vitro, élicitées dans le milieu de culture in vitro, séchées, puis broyées (éventuellement après une ou plusieurs étapes de lavage/ séchage) d'au moins une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces, et dispersées dans une composition de traitement du corps humain.

**21.** Procédé suivant la revendication précédente, **caractérisé en ce que** les cellules sont séchées par lyophilisation avant d'être soumises à un broyage.

**22.** Procédé suivant la revendication 20 ou 21, **caractérisé en ce qu'**on utilise un agent ou un moyen d'élicitation ne formant pas une impureté dans le broyat séché de cellules dédifférentiées et élicitées.

**23.** Procédé d'obtention de phytoalexine (s), dans lequel :

- on met dans un milieu de culture des cellules végétales dédifférenciées d'au moins une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces,
- après et/ou pendant la culture, on élicite les cellules dédifférentiées dans le milieu de culture,
- on sépare les cellules dédifférentiées et élicitées du milieu de culture ,
- on soumet éventuellement les cellules dédifférenciées élicitées à un ou plusieurs lavages,
- on sèche avantageusement, de préférence on lyophilise, les cellules dédifférenciées et élicitées,
- on broie les cellules dédifférenciées, élicitées de manière à former un broyat, et
- on soumet le broyat à une extraction pour extraire une ou plusieurs phytoalexines du broyat, éventuellement après une étape de remise des cellules broyées dans un milieu, en particulier un milieu aqueux et/ ou alcoolique.

**24.** Procédé suivant l'une quelconque des revendications 17 à 23, **caractérisé en ce qu'**on contrôle l'étape d'élicitation desdites cellules végétales différenciées dans leur milieu de culture in vitro, de manière à obtenir un milieu contenant au moins 0,1 % en poids de stilbènes par rapport au poids sec des cellules végétales dédifférentiées et élicitées, en particulier au moins 0,2 % en poids de stilbènes par rapport au poids sec des cellules.

**25.** Broyat de cellules végétales dédifférenciées d'au moins une espèce choisie parmi Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé et les mélanges de cellules de telles espèces, élicitées en milieu de culture in vitro, puis séchées, dans lequel ledit broyat contenant au moins une phytoalexine comprend au moins 95% en poids, avantageusement au moins 97% en poids, de préférence au moins 99% en poids de l'ensemble des matières sèches issues des cellules végétales broyées dédifférenciées et élicitées in vitro, ledit broyat étant sous une forme apte à être dispersée dans une composition cosmétique et/ ou pharmaceutique.

**26.** Broyat suivant la revendication 25, **caractérisé en ce qu'**il est exempt d'agent d'élicitation et/ou de milieu de culture.

**27.** Broyat de cellules végétales suivant la revendication 25 ou 26, **caractérisé en ce qu'**il se présente une granulométrie moyenne inférieure à 10$\mu$m.

**28.** Broyat de cellules végétales suivant l'une des revendications 25 à 27, **caractérisé en ce qu'**il contient au moins 0,1 % en poids de stilbènes par rapport au poids sec des cellules, en particulier au moins 0,2 % en poids de stilbènes par rapport au poids sec des cellules, de préférence au moins 0,5 % en poids de stilbènes par rapport au poids sec des cellules.

**29.** Composition pour application topique, en particulier composition cosmétique, contenant des cellules végétales dédifférenciées, **caractérisée en ce que** la composition contient au moins un broyat contenant au moins une phytoalexine, ledit broyat comprenant au moins 95%, avantageusement au moins 97%, de préférence au moins 99% en poids de l'ensemble des matières sèches issues des cellules végétales broyées dédifférenciées et élicitées in vitro, dans lequel le broyat contient au moins 0,1 % en poids de stilbènes par rapport au poids sec des cellules, en particulier au moins 0,2 % en poids de stilbènes par rapport au poids sec des cellules, de préférence au moins 0,5 % en poids de stilbènes par rapport au poids sec des cellules, ledit broyat étant un broyat d'une ou de cellules végétales dédifférenciées, élicitées puis séchées choisies parmi le groupe constitué des espèces suivantes : Salvia, Coleus, Rosmarinus, Ginkgo, Cannabis, Colchicum, Gloriosa, Asparagus, Arganier, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum , Iris, vigne, taxus, séquoia, chlorophytum, cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, thé, et leurs mélanges.

**Patentansprüche**

1. Zusammensetzung zur topischen Anwendung, insbesondere eine kosmetische Zusammensetzung, umfassend undifferenzierte Pflanzenzellen **dadurch gekennzeichnet, dass** die Zusammensetzung mindestens ein Zerkleinerungsprodukt von undifferenzierten und zur Synthese von mindestens einem Phytoalexin angeregten Pflanzenzellen einer in vitro Kultur von einer Art ausgewählt aus Salvia, Coleus, Rosmarinus, Gingko, Cannabis, Colchicum, Gloriosa, Asparagus, Arganbaum, Glyzinie, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, Weinrebe, Taxus, Sequoia, Chlorophytum, Cacao, Psoralea coryilfolia, Vitex negundo, Commiphora wighii, Eucalyptus punctata, Lavandula angustifolia, Citrus limon, Vanilla planifolia, Marrubium vulgare, Pilocarpus jaborandi, Rosen, Betula, Tee, und Mischungen von Zellen dieser Arten enthält,
wobei die Anregung zur Synthese von mindestens einem Phytoalexin vorteilhaft nach einem ohne Anregung durchgeführten in vitro Kulturschritt der Pflanzenzellen erfolgt, wobei das enthaltende mindestens ein Phytoalexin enthaltende Zerkleinerungsprodukt mindestens 95 Gew.-%, vorzugsweise mindestens 97 Gew.-%, mehr bevorzugt mindestens 99 Gew.-% in Bezug auf die gesamten Trockensubstanzen, umfasst, welche aus zerkleinerten undifferenzierten und in vitro angeregten Pflanzenzellen stammen, wobei das Zerkleinerungsprodukt von in der Zusammensetzung dispergiert ist oder in einer Form ist, die zum Dispergieren in die Zusammensetzung geeignet ist.

2. Zusammensetzung nach Anspruch 1, **dadurch gekennzeichnet, dass** das Zerkleinerungsprodukt von undifferenzierten und in vitro angeregten Pflanzenzellen Partikel, welche aus Vakuolen stammen, Partikel, welche aus dem Zytoplasma stammen und Teilchen, welche aus der Pektin-Cellulose Membran stammen, umfasst, wobei das Zerkleinerungsprodukt mindestens 0,1 Gew.-% Phytoalexin(e) enthält.

3. Zusammensetzung nach Anspruch 1 oder 2, **dadurch gekennzeichnet, dass** das Zerkleinerungsprodukt ein Zerkleinerungsprodukt von undifferenzierten und in vitro angeregten Zellen ist, wobei die Zellen mindestens teilweise getrocknet sind.

4. Zusammensetzung nach Anspruch 3, **dadurch gekennzeichnet, dass** das Zerkleinerungsprodukt ein Zerkleinerungsprodukt von undifferenzierten und in vitro angeregten Zellen ist, wobei die Zellen im Wesentlichen vollständig getrocknet, vorzugsweise lyophilisiert, und danach zerkleinert sind.

5. Zusammensetzung nach einem der Ansprüche 1 bis 4, **dadurch gekennzeichnet, dass** sie 0,005 bis 25 Gew.-%, vorzugsweise 0,005 bis 5 Gew.-% vom Zerkleinerungsprodukt von undifferenzierten und in vitro angeregten Pflanzenzellen enthält, wobei das Gewicht in trockener Form berechnet wird.

6. Zusammensetzung nach einem der Ansprüche 1 bis 5, **dadurch gekennzeichnet, dass** sie ein trockenes oder merklich trockenes Zerkleinerungsprodukt von undifferenzierten und in vitro angeregten Pflanzenzellen enthält, wobei das trockene oder merklich trockene Zerkleinerungsprodukt einen Wassergehalt von weniger als 25 Gew.-%, vorzugsweise von weniger als 15 Gew.-%, mehr bevorzugt von weniger als 10 Gew.-% enthält.

7. Zusammensetzung nach einem der Ansprüche 1 bis 6, **dadurch gekennzeichnet, dass** das Zerkleinerungsprodukt eine mittlere Teilchengröße der festen Partikel von weniger als 100 $\mu$m, vorzugsweise weniger als 10 $\mu$m, mehr bevorzugt weniger als 1 $\mu$m aufweist.

8. Zusammensetzung nach Anspruch 7, **dadurch gekennzeichnet, dass** die Partikel des Zerkleinerungsprodukts eine Teilchengröße aufweisen, sodass 90 Gew.-% der Partikel eine Teilchengröße im Bereich einer Teilchengröße von - 25% bis einer Teilchengröße von + 25% aufweisen.

9. Zusammensetzung nach einem der vorhergehenden Ansprüche, **dadurch gekennzeichnet, dass** das Zerkleinerungsprodukt von undifferenzierten und in vitro angeregten Pflanzenzellen mindestens ein Phytoalexin enthält, welches durch in vitro Anregung der undifferenzierten Pflanzenzellen synthetisiert wird.

10. Zusammensetzung nach einem der vorhergehenden Ansprüche, **dadurch gekennzeichnet, dass** das Zerkleinerungsprodukt von undifferenzierten und angeregten Pflanzenzellen ein Zerkleinerungsprodukt von undifferenzierten und in vitro durch ein Mittel im Kulturmedium angeregten Pflanzenzellen ist, wobei das Zerkleinerungsprodukt von dem Mittel nach Anregung merklich befreit ist.

11. Zusammensetzung nach dem vorhergehenden Anspruch, **dadurch gekennzeichnet, dass** die undifferenzierten

Zellen in vitro durch ein flüchtiges Mittel angeregt werden.

12. Zusammensetzung nach einem der vorhergehenden Ansprüche, **dadurch gekennzeichnet, dass** das Zerkleinerungsprodukt von undifferenzierten und in vitro angeregten Pflanzenzellen mindestens eine Terpen- oder Tannin- oder Polyphenol-Verbindung enthält, wobei die Verbindung durch in vitro Anregung der undifferenzierten Pflanzenzellen in ihrem Kulturmedium synthetisiert wird.

13. Zusammensetzung nach einem der vorhergehenden Ansprüche, **dadurch gekennzeichnet, dass** das Zerkleinerungsprodukt von undifferenzierten und in vitro angeregten Pflanzenzellen in Form einer viskosen Suspension oder eines Gels oder in einem merklich trockenen Pulver vorliegen, wobei die Suspension, das Gel oder das Pulver in einer Form vorliegt, die vorzugsweise zum Dispergieren in die Zusammensetzung geeignet ist.

14. Zusammensetzung nach einem der vorhergehenden Ansprüche, **dadurch gekennzeichnet, dass** das Zerkleinerungsprodukt der Zellen ein Zerkleinerungsprodukt von undifferenzierten und in vitro angeregten Weinrebenzellen ist.

15. Zusammensetzung nach einem der vorhergehenden Ansprüche, **dadurch gekennzeichnet, dass** sie ein Zerkleinerungsprodukt von undifferenzierten, kultivierten und in ihrem Kulturmedium in vitro angeregten Zellen enthält, wobei das Zerkleinerungsprodukt vorzugsweise vom Kulturmedium merklich befreit ist.

16. Zusammensetzung nach einem der vorhergehenden Ansprüche, **dadurch gekennzeichnet, dass** sie ein Zerkleinerungsprodukt von undifferenzierten und in vitro angeregten Zellen enthält, wobei das Zerkleinerungsprodukt mindestens 0,1 Gew.-% von Stilbenen in Bezug auf das Trockengewicht der undifferenzierten angeregten zerkleinerten Zellen, vorzugsweise mindestens 0,2 Gew.-% von Stilbenen in Bezug auf das Trockengewicht der undifferenzierten angeregten zerkleinerten Zellen, mehr bevorzugt mindestens 0,5 Gew.-% von Stilbenen in Bezug auf das Trockengewicht der undifferenzierten angeregten zerkleinerten Zellen enthält.

17. Verfahren zur Herstellung einer Zusammensetzung zur topischen Anwendung nach einem der vorhergehenden Ansprüche, **dadurch gekennzeichnet, dass** die undifferenzierten Pflanzenzellen von mindestens einer Art ausgewählt aus Salvia, Coleus, Rosmarinus, Gingko, Cannabis, Colchicum, Gloriosa, Asparagus, Arganbaum, Glyzinie, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, Weinrebe, Taxus, Sequoia, Chlorophytum, Cacao, Psoralea coryilfolia, Vitex negundo, Commiphora wighii, Eucalyptus punctata, Lavandula angustifolia, Citrus limon, Vanilla planifolia, Marrubium vulgare, Pilocarpus jaborandi, Rosen, Betula, Tee, und Mischungen von Zellen dieser Arten in ein Kulturmedium gesetzt werden, sodass das Wachstum der Zellen ermöglicht wird, dass die differenzierten Pflanzenzellen in ihrem Kulturmedium in einem ausreichenden Zeitraum angeregt werden, zur Synthese einer ausreichenden Menge von Metaboliten, und dass die in einem Kulturmedium angeregten Pflanzenzellen mit einem oder mehreren Hilfsstoffen gemischt werden zur Herstellung einer kosmetischen Zusammensetzung, wobei die Zellen vor und/oder nach ihrer Mischung mit einem oder mehreren Hilfsstoffen und/oder vor und/oder nach einer Trockenstufe zerkleinert werden.

18. Verfahren nach Anspruch 17, **dadurch gekennzeichnet, dass** die in vitro angeregten Zellen einem Trocknen gefolgt von einem Zerkleinern unterworfen werden.

19. Verfahren nach einem der Ansprüche 17 oder 18, **dadurch gekennzeichnet, dass** die Zellen in ihrem in vitro Kulturmedium mithilfe eines Mittels angeregt werden, welches sich nach Extraktion der angeregten Zellen vom Kulturmedium nicht in den angeregten Zellen befindet, wobei die Membranstruktur der Zellen beibehalten wird.

20. Verfahren, wobei die undifferenzierten Pflanzenzellen in in vitro Kultur gesetzt werden, im in vitro Kulturmedium angeregt werden, getrocknet, dann zerkleinert (gegebenenfalls nach einer oder mehreren Wasch-/Trockenstufen) von mindestens einer Art ausgewählt aus Salvia, Coleus, Rosmarinus, Gingko, Cannabis, Colchicum, Gloriosa, Asparagus, Arganbaum, Glyzinie, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, Weinrebe, Taxus, Sequoia, Chlorophytum, Cacao, Psoralea coryilfolia, Vitex negundo, Commiphora wighii, Eucalyptus punctata, Lavandula angustifolia, Citrus limon, Vanilla planifolia, Marrubium vulgare, Pilocarpus jaborandi, Rosen, Betula, Tee, und Mischungen von Zellen dieser Arten, und in eine Zusammensetzung zur Behandlung des menschlichen Körpers dispergiert werden.

21. Verfahren nach dem vorhergehenden Anspruch, **dadurch gekennzeichnet, dass** die Zellen durch Lyophilisation

getrocknet werden, bevor sie einem Zerkleinern unterworfen werden.

**22.** Verfahren nach Anspruch 20 oder 21, **dadurch gekennzeichnet, dass** ein Mittel oder ein Anregungsmittel verwendet wird, welches keine Verunreinigung in dem trocknen Zerkleinerungsprodukt undifferenzierter und angeregter Zellen bildet.

**23.** Verfahren zum Erhalten von Phytoalexin(en), wobei:

- undifferenzierte Pflanzenzellen von mindestens von mindestens einer Art ausgewählt aus Salvia, Coleus, Rosmarinus, Gingko, Cannabis, Colchicum, Gloriosa, Asparagus, Arganbaum, Glyzinie, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, Weinrebe, Taxus, Sequoia, Chlorophytum, Cacao, Psoralea coryilfolia, Vitex negundo, Commiphora wighii, Eucalyptus punctata, Lavandula angustifolia, Citrus limon, Vanilla planifolia, Marrubium vulgare, Pilocarpus jaborandi, Rosen, Betula, Tee, und Mischungen von Zellen dieser Arten in ein Kulturmedium gesetzt werden,
- nach und/oder während der Kultivierung die undifferenzierten Zellen in dem Kulturmedium angeregt werden,
- die undifferenzierten und angeregten Zellen vom Kulturmedium getrennt werden,
- gegebenenfalls die undifferenzierten angeregten Zellen einer oder mehreren Waschstufen unterworfen werden,
- die undifferenzierten und angeregten Zellen vorteilhafterweise getrocknet, vorzugsweise lyophilisiert werden,
- die undifferenzierten angeregten Zellen zerkleinert werden, um ein Zerkleinerungsprodukt zu bilden, und
- das Zerkleinerungsprodukt einer Extraktion unterworfen wird, zur Extraktion eines oder mehreren Phytoalexinen aus dem Zerkleinerungsprodukt, gegebenenfalls nach einem Schritt des Versetzens der zerkleinerten Zellen mit einem Medium, insbesondere einem wässrigen und/oder alkoholischen Medium.

**24.** Verfahren nach einem der Ansprüche 17 bis 23, **dadurch gekennzeichnet, dass** der Anregungsschritt der differenzierten Pflanzenzellen in ihrem in vitro Kulturmedium kontrolliert wird, um ein Medium zu erhalten, welches mindestens 0,1 Gew.-% von Stilbenen in Bezug auf das Trockengewicht der undifferenzierten und angeregten Pflanzenzellen, vorzugsweise mindestens 0,2 Gew.-% von Stilbenen in Bezug auf das Trockengewicht der Zellen enthält.

**25.** Zerkleinerungsprodukt von undifferenzierten Pflanzenzellen von mindestens einer Art ausgewählt aus Salvia, Coleus, Rosmarinus, Gingko, Cannabis, Colchicum, Gloriosa, Asparagus, Arganbaum, Glyzinie, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, Weinrebe, Taxus, Sequoia, Chlorophytum, Cacao, Psoralea coryilfolia, Vitex negundo, Commiphora wighii, Eucalyptus punctata, Lavandula angustifolia, Citrus limon, Vanilla planifolia, Marrubium vulgare, Pilocarpus jaborandi, Rosen, Betula, Tee, und Mischungen von Zellen dieser Arten, welche in einem in vitro Kulturmedium angeregt werden, dann getrocknet, wobei das Zerkleinerungsprodukt von enthaltend mindestens ein Phytoalexin mindestens 95 Gew.-%, vorzugsweise mindestens 97 Gew.-%, mehr bevorzugt mindestens 99 Gew.-% in Bezug auf das Gewicht der gesamten Trockensubstanzen umfasst, welches aus den zerkleinerten undifferenzierten und in vitro angeregten Pflanzenzellen stammt, wobei das Zerkleinerungsprodukt in einer Form ist, die zum Dispergieren in eine kosmetische und/oder pharmazeutische Zusammensetzung geeignet ist.

**26.** Zerkleinerungsprodukt nach Anspruch 25, **dadurch gekennzeichnet, dass** es von Anregungsmittel und/oder Kulturmedium befreit ist.

**27.** Zerkleinerungsprodukt von Pflanzenzellen nach Anspruch 25 oder 26, **dadurch gekennzeichnet, dass** es eine mittlere Teilchengröße von weniger als 10 $\mu$m aufweist.

**28.** Zerkleinerungsprodukt von Pflanzenzellen nach einem der Ansprüche 25 bis 27, **dadurch gekennzeichnet, dass** es mindestens 0,1 Gew.-% von Stilbenen in Bezug auf das Trockengewicht der Zellen, vorzugsweise mindestens 0,2 Gew.-% von Stilbenen in Bezug auf das Trockengewicht der Zellen, mehr bevorzugt mindestens 0,5 Gew.-% von Stilbenen in Bezug auf das Trockengewicht der Zellen enthält.

**29.** Zusammensetzung zur topischen Anwendung, insbesondere eine kosmetische Zusammensetzung, enthaltend undifferenzierte Pflanzenzellen, **dadurch gekennzeichnet, dass** die Zusammensetzung mindestens ein Zerkleinerungsprodukt enthaltend mindestens ein Phytoalexin enthält, wobei das Zerkleinerungsprodukt mindestens 95 Gew.-

%, vorzugsweise mindestens 97 Gew.-%, mehr bevorzugt mindestens 99 Gew.-% in Bezuf auf die gesamten Trockensubstanzen umfasst, welches aus den zerkleinerten undifferenzierten und in vitro angeregten Pflanzenzellen stammt, wobei das Zerkleinerungsprodukt mindestens 0,1 Gew.-% von Stilbenen in Bezug auf das Trockengewicht der Zellen, vorzugsweise mindestens 0,2 Gew.-% von Stilbenen in Bezug auf das Trockengewicht der Zellen, mehr bevorzugt mindestens 0,5 Gew.-% von Stilbenen in Bezug auf das Trockengewicht der Zellen enthält, wobei das Zerkleinerungsprodukt ein Zerkleinerungsprodukt von einer oder mehreren undiffererizierten, angeregten dann getrockneten Pflanzenzellen ausgewählt aus der Gruppe bestehend aus den folgenden Arten: Salvia, Coleus, Rosmarinus, Gingko, Cannabis, Colchicum, Gloriosa, Asparagus, Arganbaum, Glyzinie, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, Weinrebe, Taxus, Sequoia, Chlorophytum, Cacao, Psoralea Coryilfolia, Vitex Negundo, Commiphora Wighii, Eucalyptus Punctata, Lavandula Angustifolia, Citrus Limon, Vanilla Planifolia, Marrubium Vulgare, Pilocarpus Jaborandi, Rosen, Betula, Tee, und ihren Mischungen, ist.

## Claims

1. A composition for topical application, especially a cosmetic composition, containing dedifferentiated plant cells, **characterised in that** the composition contains at least a communited product of dedifferentiated plant cells elicited in vitro in a culture of a species selected from Salvia, Coleus, Rosmarinus, Gingko, Cannabis, Colchicum, Gloriosa, Asparagus, Arganier, Wisteria, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vine, taxus, blue lotus, oriental cherry, sequoia, chlorophytum, Cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, tea and mixtures of cells of such species, in order to synthesise at least one phytoalexin, said elicitation for synthesising at least one phytoalexin being advantageously operated after a in vitro culture step of the plant cells without elicitation, in which the communited product containing at least one phytoalexin comprises at least 95%, advantageously at least 97%, preferably at least 99% by weight of the entirety of the dry materials derived from the in vitro elicited and dedifferentiated comminuted plant cells, the communited product being dispersed or in a form suitable for being dispersed in said composition.

2. The composition according to claim 1, **characterised in that** the communited product of in vitro elicited and dedifferentiated plant cells comprises particles derived from vacuoles, particles derived from cytoplasm, and particles derived from pecto-cellulose membrane, said comminuted product containing at least 0.1% by weight of phytoalexin(s).

3. The composition according to claim 1 or 2, **characterised in that** the comminuted product is a comminuted product of in vitro elicited and dedifferentiated cells, said cells being at least partially dried.

4. The composition according to claim 3, **characterised in that** the comminuted product is a comminuted product of in vitro elicited and dedifferentiated cells, said cells being substantially completely dried, preferably freeze-dried, and then comminuted.

5. The composition according to any one of the claims 1 to 4, **characterised in that** it contains 0.005 to 25% by weight, advantageously 0.005 to 5% by weight of a comminuted product of in vitro elicited and dedifferentiated plant cells, said weight being calculated in dry form.

6. The composition according to anyone of the claims 1 to 5, **characterised in that** it contains a dry or substantially dry comminuted product of in vitro elicited and dedifferentiated plant cells, said dry or substantially dry comminuted product having a water content less than 25% by weight, advantageously less than 15% by weight, preferably less than 10% by weight.

7. The composition according to anyone of the claims 1 to 6, **characterised in that** the communited product has an average solid particle size of less than 100 μm, advantageously less than 10μm, preferably less than 1μm.

8. The composition according to claim 7, **characterised in that** the particles of the communited product have a particle size such that 90% by weight of the particles have a particle size ranging from the average particle size - 25% to the average particle size + 25%.

**9.** The composition according to anyone of the preceding claims, **characterised in that** said comminuted product of in vitro elicited and dedifferentiated plant cells contains at least one phytoalexin synthesised by the in vitro elicitation of dedifferentiated plant cells.

**10.** The composition according to anyone of the preceding claims, **characterised in that** said comminuted product of in vitro elicited and dedifferentiated plant cells is a comminuted product of in vitro elicited and dedifferentiated plant cells by an agent in the culture medium, said comminuted product being substantially free from said agent after elicitation.

**11.** The composition according to the preceding claim, **characterised in that** the dedifferentiated cells are elicited in vitro by a volatile agent.

**12.** The composition according to anyone of the preceding claims, **characterised in that** said comminuted product of in vitro elicited and dedifferentiated plant cells contains at least one terpenic or tannic or polyphenolic compound, said compound being synthesised by the in vitro elicitation of the dedifferentiated plant cells in their culture medium.

**13.** The composition according to anyone of the preceding claims, **characterised in that** the comminuted product of in vitro elicited and dedifferentiated plant cells is in the form of a viscous suspension or a gel or a substantially dry powder, said suspension, gel or powder being advantageously in a form suitable to be dispersed in said composition.

**14.** The composition according to anyone of the preceding claims, **characterised in that** the comminuted product is a comminuted product of dedifferentiated vine cells which are elicited in vitro.

**15.** The composition according to anyone of the preceding claims, **characterised in that** it contains a comminuted product of dedifferentiated cells, growth and elicited in their in vitro culture medium, said comminuted product being advantageously substantially free from the culture medium.

**16.** The composition according to anyone of the preceding claims, **characterised in that** it contains a comminuted product of in vitro elicited and dedifferentiated plant cells, said comminuted product containing at least 0.1% by weight of stilbenes based on the dry weight of the comminuted, dedifferentiated, elicited cells, especially at least 0.2% by weight of stilbenes based on the dry weight of the comminuted, dedifferentiated, elicited cells, preferably at least 0.5% by weight of stilbenes based on the dry weight of the comminuted, dedifferentiated, elicited cells.

**17.** A method of preparing a composition for topical use according to anyone of the preceding claims, **characterised in that** dedifferentiated plant cells of at least one species selected from Salvia, Coleus, Rosmarinus, Gingko, Cannabis, Colchicum, Gloriosa, Asparagus, Arganier, Wisteria, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vine, taxus, blue lotus, oriental cherry, sequoia, chlorophytum, Cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, tea and mixtures of cells of such species, are placed in a culture medium so as to enable a growth of the cells in an in vitro culture medium, **in that** the dedifferentiated plant cells are elicited in their culture medium during a period of time sufficient for the synthesis of a sufficient quantity of metabolites, and **in that** the plant cells elicited in the in vitro culture medium are mixed with one or more excipients in order to prepare a cosmetic composition, the cells being comminuted before and/or after their mixing with one or more excipients, and/or before and/or after a drying step.

**18.** The method according to claim 17, **characterised in that** said cells elicited in vitro are subjected to a drying step, followed by comminution.

**19.** The method according to anyone of the claims 17 and 18, **characterised in that** the cells are elicited in their in vitro culture medium by means of an agent which, after extracting the elicited cells from the culture medium whilst retraining the membrane structure of the cells, does not occur in the elicited cells.

**20.** The method in which the dedifferentiated plant cells of at least one species receding claims, **characterised in that** dedifferentiated plant cells of at least one species selected from Salvia, Coleus, Rosmarinus, Gingko, Cannabis, Colchicum, Gloriosa, Asparagus, Arganier, Wisteria, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vine, taxus, blue lotus, oriental cherry, sequoia, chlorophytum, Cacao, psoralea coryilfolia, vitex

negundo, commiphora wighii, eucalyptus punctata, lavandula angustifolia, citrus limon, vanilla planifolia, marrubium vulgare, pilocarpus jaborandi, roses, betula, tea and mixtures of cells of such species are placed in a growth culture in vitro, are elicited in said in vitro culture medium, are dried , are then comminuted (possibly after one or more washing/drying steps) and dispersed in a composition for the treatment of the human body.

**21.** The method according to claim 20, in which the cells are dried by freeze-drying before being communited.

**22.** The method according to claim 20 or 21, **characterised in that** an eliciting agent or means which does not form an impurity in the comminuted product of dedifferentiated and elicited cells, is used.

**23.** The method for obtaining phytoalexin(s), in which :

- dedifferentiated plant cells of at least one species selected from Salvia, Coleus, Rosmarinus, Gingko, Cannabis, Colchicum, Gloriosa, Asparagus, Arganier, Wisteria, Medicago, Mungo, Erythrina, Oenothera, Papaver, Atropa, Datura, Solanum, Borago, Reseda, Amsonia, Catharantus, Pilocarpus; Digitalis, Coffea, Theobroma, Jasminum, Capsicum, Iris, vine, taxus, blue lotus, oriental cherry, sequoia, chlorophytum, Cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, tea and mixtures of cells of such species, are placed in a culture medium,
- the dedifferentiated cells are elicited in the culture medium, after and/or during the culture,
- the dedifferentiated and elicited cells are separated from the culture medium,
- the dedifferentiated and elicited cells are possibly subjected to one or more washing steps,
- the dedifferentiated and elicited cells are dried, advantageously freeze dried,
- the dedifferentiated and elicited cells are comminuted in order to form a comminuted product, and
- the comminuted product is subjected to an extraction to extract one or more phytoalexins from the comminuted product, possibly after a step of introducing the communited cells in a medium, especially an aqueous and/or alcoholic medium.

**24.** The method according to anyone of the claims 17 to 23, **characterised in that** the stage of eliciting said dedifferentiated plant cells in their in vitro culture medium is controlled in order to obtain a medium containing at least 0.1% by weight of stilbenes based on the dry weight of the dedifferentiated, elicited plant cells, especially at least 0.2% by weight of stilbenes based on the dry weight of the cells.

**25.** A comminuted product of dedifferentiated plant cells of at least one species selected from Salvia, Coleus, Rosmarinus, Gingko, Cannabis, Colchicum, Gloriosa, Asparagus, Arganier, Wisteria, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum, Iris, vine, taxus, blue lotus, oriental cherry, sequoia, chlorophytum, Cacao, psoralea coryilfolia, vitex negundo, commiphora wighii, eucalyptus punctata, lavandula angustifolia, citrus limon, vanilla planifolia, marrubium vulgare, pilocarpus jaborandi; roses, betula, tea and mixtures of cells of such species, which are elicited in an in vitro culture medium and then dried, wherein said comminuted product containing at least one phytoalexin comprises at least 95% by weight, advantageously at least 97% by weight, preferably at least 99% by weight of the entirety of the dry materials derived from the dedifferentiated plant cells which are elicited in vitro, said comminuted product being in a form suitable for being dispersed in a cosmetic and/or pharmaceutical composition.

**26.** The communited product according to claim 25, **characterised in that** it is free from eliciting agent and/or culture medium.

**27.** The communited product of plant cells according to claim 25 or 26, **characterised in that** it has an average particle size of less than 10μm.

**28.** The communited product of plant cells according to anyone of the claims 25 to 27, **characterised in that** it contains at least 0.1% by weight of stilbenes based on the dry weight of the cells, especially at least 0.2% by weight of stilbenes based on the dry weight of the cells, preferably at least 0.5% by weight of stilbenes based on the dry weight of the cells.

**29.** A composition for topical application, especially a cosmetic composition, containing dedifferentiated plant cells, **characterized in that** the composition contains at least a communited product containing at least one phytoalexin, said communited product containing at least one phytoalexin comprises at least 95% by weight, advantageously at

least 97% by weight, preferably at least 99% by weight of the entirety of the dry materials derived from the dedifferentiated plant cells which are elicited in vitro, in which the communited product contains at least 0.1% by weight of stilbenes based on the dry weight of the cells, especially at least 0.2% by weight of stilbenes based on the dry weight of the cells, preferably at least 0.5% by weight of stilbenes based on the dry weight of the cells, said communited product being a communited product of one or more dedifferentiated plant cells, elicited and then dried selected from the group consisting of the following species : Salvia, Coleus, Rosmarinus, Gingko, Cannabis, Colchicum, Gloriosa, Asparagus, Arganier, Wisteria, Medicago, Mungo, Erythrina, Oenothera, Papaver, Atropa, Datura, Solanum, Borago, Reseda, Amsonia, Catharantus, Pilocarpus, Digitalis; Coffea, Theobroma, Jasminum, Capsicum, Iris, vine, taxus, blue lotus, oriental cherry, sequoia, chlorophytum, Cacao, psoralea coryilfolia, vitex negundo, commiphora wighii, eucalyptus punctata, lavandula angustifolia, citrus limon, vanilla planifolia, marrubium vulgare, pilocarpus jaborandi, roses, betula, tea and mixtures of cells of such species.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2795637
- US 4241536 A
- EP 378921 A
- WO 8800968 A
- EP 1203811 A
- FR 2808190


### Littérature non-brevet citée dans la description


- **E.F. GEORGE ; P.D. SHERRINGTON.** Plant Propagation by tissue culture, handbook and directory of commercial laboratories. Exegetics Ltd, 1984
- **E.F. GEORGE ; DJM PUTTOCK ; H.J GEORGE.** Plant Culture Média : formulations and uses. Exegetics Ltd, 1987
- **DOUILLET-BREUIL et al.** Changes in the phytoalexin content of various Vitis spp. in response to ultraviolet C elicitation. *Journal of Agricultural and Food chemistry, US,* Octobre 1999, vol. 47 (10